# EUROPEAN PATENT APPLICATION

(11) **EP 1 669 451 A1**
(43) Date of publication of application: **14.06.2006**
(21) Application number: 04730714.5
(22) Date of filing: 30.04.2004
(51) Int. Cl.: C12N 15/12, C12N 15/09, C07K 14/47, C12Q 1/68, G01N 33/50

(54) **BRAIN TUMOR MARKER AND METHOD OF DIAGNOSING BRAIN TUMOR**

(30) Priority: 01.09.2003 JP 2003309356
(71) Applicant: Japan Science and Technology Agency, Kawaguchi-shi, Saitama 332-0012 (JP)
(72) Inventor: IWADATE, Yasuo, Sakura-shi, Chiba 2850837 (JP); HIWASA, Takaki, Chiba-shi, Chiba 2600808 (JP); TAKIGUCHI, Masaki, Funabashi-shi, Chiba 2740824 (JP); YAMAURA, Akira, Chosei-gun, Chiba 2970201 (JP)
(74) Representative: Soames, Candida Jane
(86) International application number: PCT/JP2004/006345
(87) International publication number: WO 2005/024023

(57) **Abstract**

The present invention provides a brain tumor marker, **characterized in that** the brain tumor marker is at least one protein expressed in a brain tumor tissue and selected from the group of proteins having the amino acid sequences represented by SEQ ID NOS: 1 to 16, respectively, wherein the protein is expressed in at least one tissue selected from the group of the tissues of a highly malignant brain tumor, a low malignant brain tumor, a drug-sensitive brain tumor, and a drug-resistant brain tumor; a polynucleotide encoding the brain tumor marker; and a method of diagnosing brain tumors by using the brain tumor marker or the polynucleotide as an indicator.

## Description

### Background of the Invention

### Technical Field

The invention of the present application relates to a marker protein useful for diagnosis of human brain tumors with respect to grade of malignancy and drug sensitivity or resistance, a polynucleotide encoding the marker protein, and a method of cancer diagnosis for brain tumors by using the marker protein or the polynucleotide.

### Background Art

Astrocytoma is diversified so widely as to make it very difficult how it should be classified pathologically. Even if astrocytomas are of a similar low grade, some of them may progress into brain tumor (glioblastoma), while others may remain in a silent state over years. Some genetic abnormalities and variation in gene expression levels have been found to be associated with tumor formation of astrocytoma and its aggravation of malignancy (Cavenee, W. K. et al., "Diffuse infiltrating astrocytomas", In: W. K. Cavenee and P. Kleihues (eds.), pp. 9-15, Lyon: IARC Press, 2000), but these events have had a limited utility in molecular biological diagnosis of glioma. Establishment of a diagnostic method based on molecular biology to predict the prognosis and therapeutic response of patients requires a more comprehensive characterization of such gene expressions as would be correlated to clinical behaviors of glioma. Molecular biological diagnosis of human glioma as described above will finally lead to a target-specific therapeutic approach to develop a therapeutic strategy for individual patients and thus enhance clinical therapeutic effects on the whole patients.

In several types of human cancer, molecular expressions have been profiled using microarrays based on oligonucleotides or cDNAs to obtain molecular markers useful for making a choice among the therapies (Alizadeh, A. A. et al., Nature 403: 503-11, 2000; and Bittner, M. et al., Nature 406: 536-540, 2000). The profiles of gene expression created using the microarray technique have been examined to reveal that many genes are expressed differentially among various histologic types or grades of glioma in the brain (Pomeroy, S. L. et al., Nature 415: 436-42, 2002; Rickman, D. S. et al., Cancer Res. 61: 6885-6891, 2001; and Sallinen, S. L. et al., Cancer Res. 60: 6617-22, 2000). However, since biological systems involve transcriptional and posttranscriptional control, posttranslational modification, and protein migration between different cell compartments, such characteristics cannot be identified by microarray systems on the level of RNA. Consequently, some molecular biological diagnoses have also been proposed, wherein their targets are proteins expressed specifically in tumor tissues including brain tumors (Japanese Patent Publication (Kokai) No. 2002-223765; and Japanese Patent Publication (Kohyo) Nos. 2003-506100, 2002-519702 and 2002-509734).

The molecular biological method of diagnosis using protein markers expressed specifically in brain tumor tissues, as described above, has been indicated to be effective to diagnose brain tumors in their early stage, and a number of novel protein markers have been proposed for the method. In order to improve the method in diagnostic accuracy, however, it is critical to provide many protein markers which reflect the biological characteristics of brain tumors or the like, and use the protein markers in combination.

It is an object of the invention of the present application to provide a novel protein marker useful for a more accurate diagnosis of brain tumors, in view of the circumstances as described above.

It is another object of the invention of the present application to provide a genetic material encoding the protein marker, an antibody against the protein marker, and a method of diagnosing brain tumors using the protein marker or antibody.

### Summary of the Invention

The present application provides the following inventions (1) to (19) in order to solve the above problems:
(1) a brain tumor marker, characterized in that the brain tumor marker is at least one protein expressed in a brain tumor tissue and selected from the group of proteins having the amino acid sequences represented by SEQ ID NOS: 1 to 16, respectively, wherein the protein is expressed in at least one tissue selected from the group of the tissues of a highly malignant brain tumor, a low malignant brain tumor, a drug-sensitive brain tumor, and a drug-resistant brain tumor;
(2) a highly malignant brain tumor marker, wherein the brain tumor marker is at least one protein selected from the group of proteins expressed in a highly malignant brain tumor tissue, wherein the protein has the amino acid sequence represented by SEQ ID NO: 1, 4, 5, 6 or 7;
(3) a low malignant brain tumor marker, wherein the brain tumor marker is at least one protein selected from the group of proteins expressed in a low malignant brain tumor tissue, wherein the protein has the amino acid sequence represented by SEQ ID NO: 2 or 3;
(4) a drug-sensitive brain tumor marker, wherein the brain tumor marker is at least one protein selected from the group of proteins expressed in a drug-sensitive brain tumor tissue, wherein the protein has the amino acid sequence represented by SEQ ID NO: 5, 8, 11, 13, 14, 15 or 16;
(5) a drug-resistant brain tumor marker, wherein the brain tumor marker is at least one protein selected from the group of proteins expressed in a drug-resistant brain tumor tissue, wherein the protein has the amino acid sequence represented by SEQ ID NO: 1, 2, 9, 10, 12 or 16;
(6) an antibody which binds to a protein selected from the group of proteins having the amino acid sequences represented by SEQ ID NOS: 1 to 16, respectively;
(7) a diagnostic kit, characterized in that the diagnostic kit comprises at least one of the antibody according to the invention (6) and/or a labeled antibody derived therefrom;
(8) a polynucleotide encoding the highly malignant brain tumor marker according to the invention (2);
(9) a polynucleotide encoding the low malignant brain tumor marker according to the invention (3);
(10) a polynucleotide encoding the drug-sensitive brain tumor marker according to the invention (4);
(11) a polynucleotide encoding the drug-resistant brain tumor marker according to the invention (5);
(12) a DNA fragment consisting of a partial contiguous sequence of any one of SEQ ID NOS: 17 to 32;
(13) a DNA microarray comprising the polynucleotide according to any one of the inventions (8) to (11) and/or the DNA fragment according to the invention (12);
(14) a set of primers for amplifying by PCR any one of the nucleotide sequences of SEQ ID NOS: 17 to 32;
(15) a method of diagnosing a highly malignant brain tumor, characterized in that a biological sample from a subject is assayed for the expression level of the polynucleotide according to the invention (8), wherein the subject is judged to be a patient with a highly malignant brain tumor if the subject has a higher expression level of at least one said polynucleotide than a healthy person;
(16) a method of diagnosing a low malignant brain tumor, characterized in that a biological sample from a subject is assayed for the expression level of the polynucleotide according to the invention (9), wherein the subject is judged to be a patient with a low malignant brain tumor if the subject has a higher expression level of at least one said polynucleotide than a healthy person;
(17) a method of diagnosing a drug-sensitive brain tumor, characterized in that a biological sample from a subject is assayed for the expression level of the polynucleotide according to the invention (10), wherein the subject is judged to be a patient with a drug-sensitive brain tumor if the subject has a higher expression level of at least one said polynucleotide than a healthy person;
(18) a method of diagnosing a drug-resistant brain tumor, characterized in that a biological sample from a subject is assayed for the expression level of the polynucleotide according to the invention (11), wherein the subject is judged to be a patient with a drug-resistant brain tumor if the subject has a higher existence level of at least one said polynucleotide than a healthy person; and
(19) a method of diagnosing a brain tumor, characterized in that said method combines the respective methods according to the inventions (15) to (18), wherein a brain tumor which a subject suffers from is judged to be one selected from the group of:
   (a) highly malignant and drug-sensitive;
   (b) highly malignant and drug-resistant;
   (c) low malignant and drug-sensitive; and
   (d) low malignant and drug-resistant.

### Brief Description of the Drawings

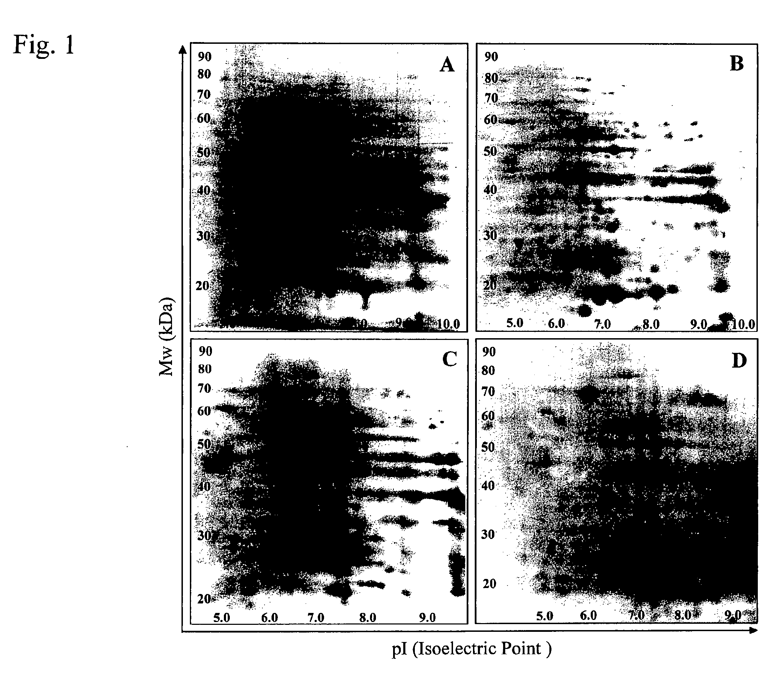

Figure 1 shows representative two-dimensional electrophoresis images of normal brain tissue (A), astrocytoma (B), undifferentiated astrocytoma (C) and multiform glioblastoma (D);

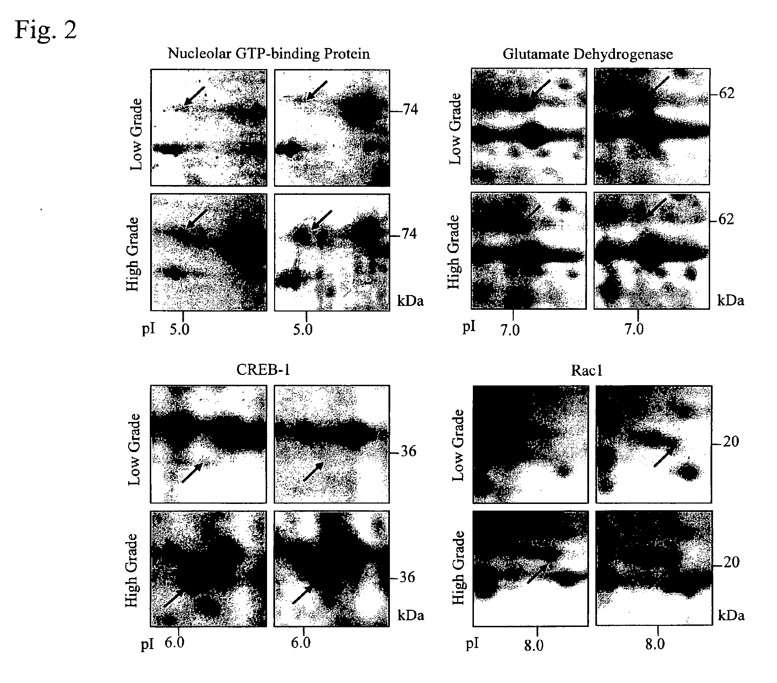

Figure 2 shows 4 different protein spots on two-dimensional electrophoresis gel, wherein the spots were of proteins expressed differently between low grade astrocytoma and high grade astrocytoma (multiform glioblastoma or undifferentiated astrocytoma);

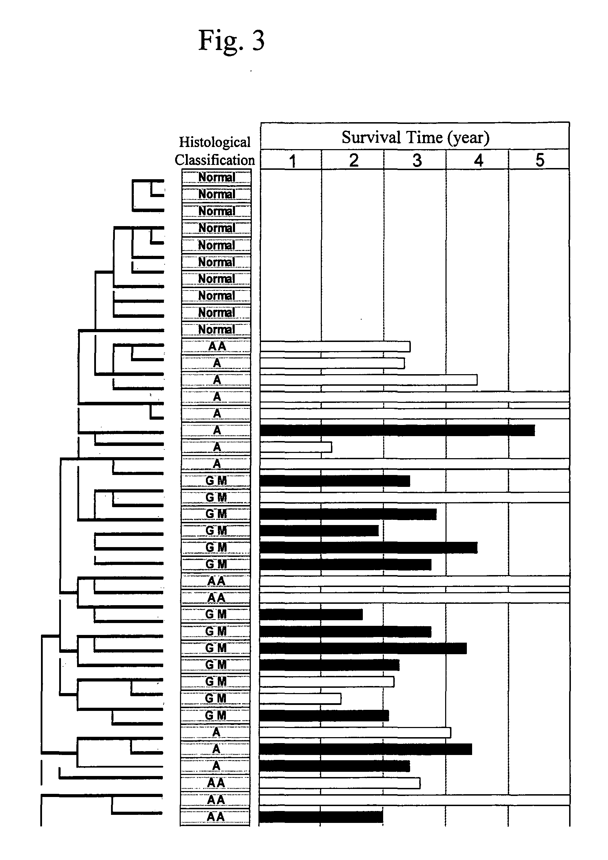

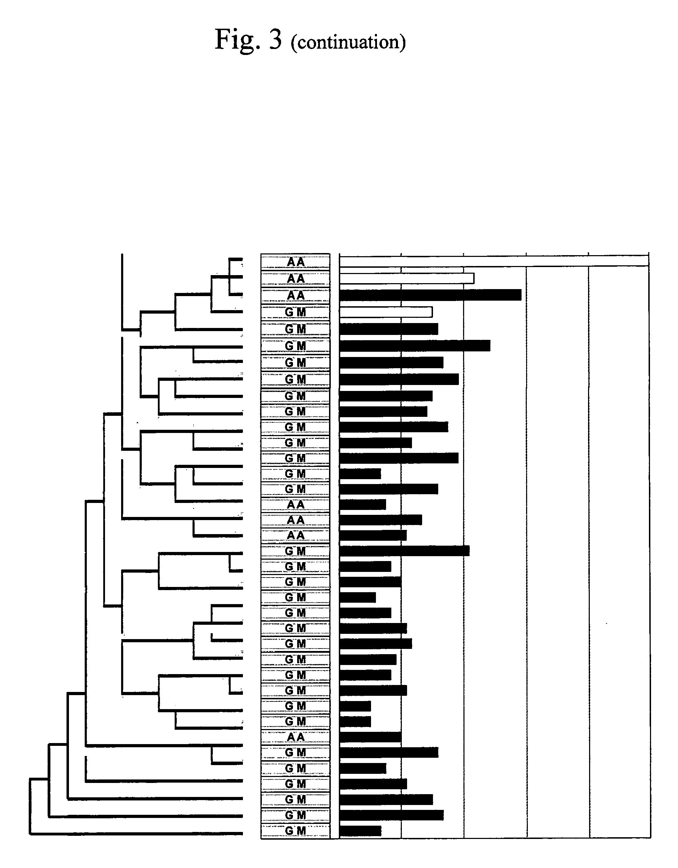

Figure 3 shows a diagram of hierarchical clustering applied on each of the cases based on proteome profiling data determined for 75 glioblastoma samples and 10 normal brain samples. The hierarchical grade was classified into 4 groups consisting of normal brain tissue (Normal), astrocytoma (A), undifferentiated astrocytoma (AA) and multiform glioblastoma (GM). Survival time of each patient is shown in the right column with a limit of 5 years at most;

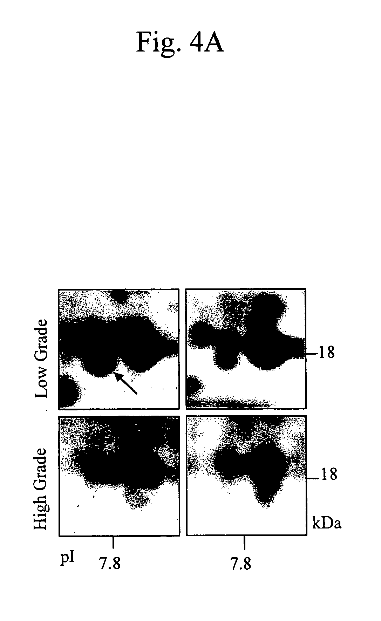

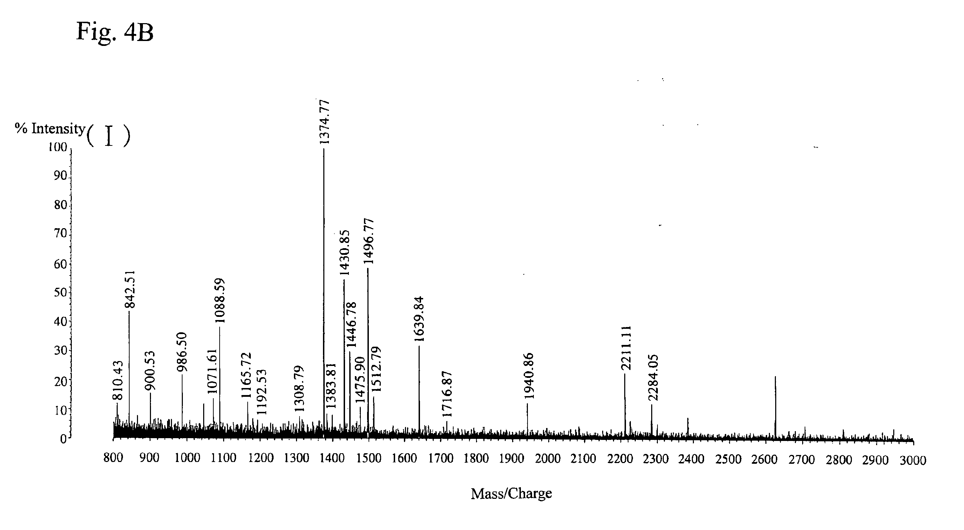

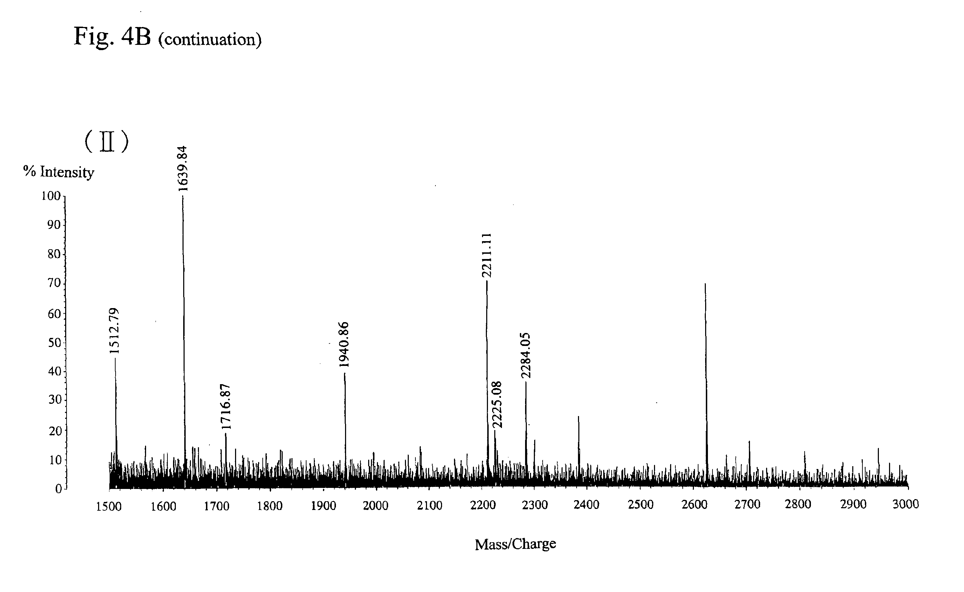

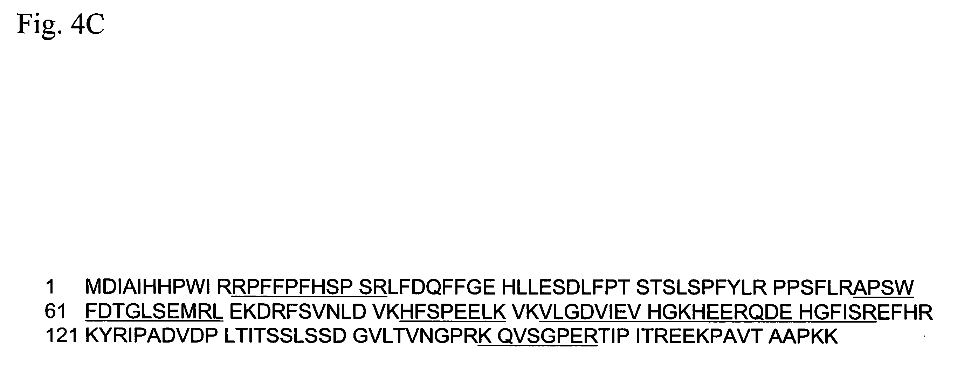

Figure 4 shows the spots of proteins differently expressed between low grade astrocytoma and high grade astrocytoma (Figure 4A), the results of analyses by matrix-associated laser desorption/ionization time-of-flight mass spectrometry (MALDI-TOF MS) for the expressed proteins (Figure 4B, (I) and (II)), and the determined amino acid sequence (Figure 4C), respectively. The determined amino acid sequence is shown in Figure 4C by underlining on the full length sequence of protein (α-crystallin);

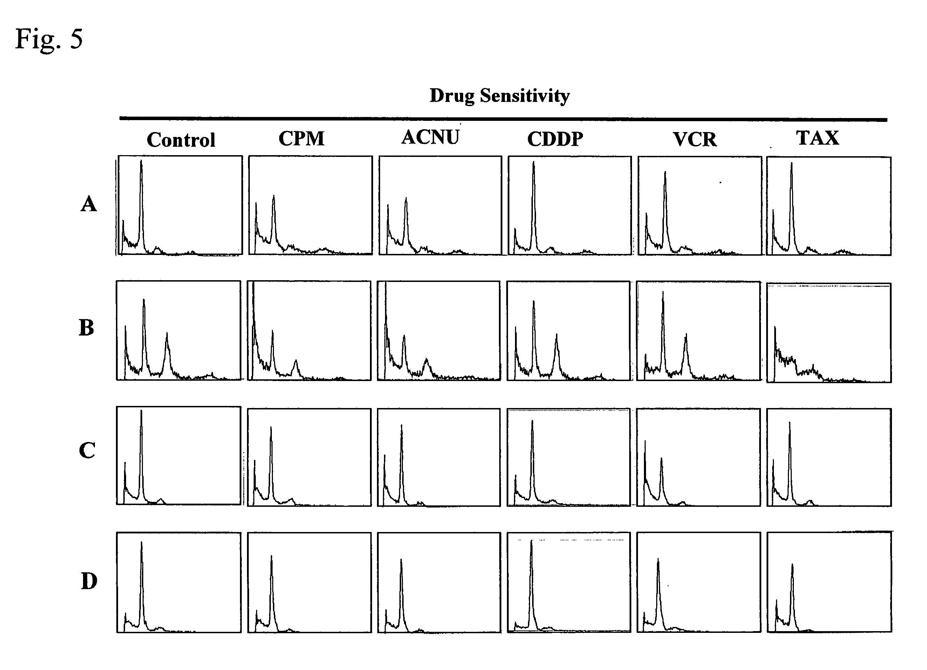

Figure 5 shows specific examples of drug sensitivity test for representative anti-cancer agents. CPM and ACNU are effective in A; CPM, ACNU and TAX are effective in B; VCR is effective in C; and D is an example in which no drug is effective. In the phylogenetic tree in sample axis, A and B are located at sites approximate to normal brain tissue, C is located at a site slightly away from normal brain tissue, and D is located at a site more far-off from normal brain tissue; and

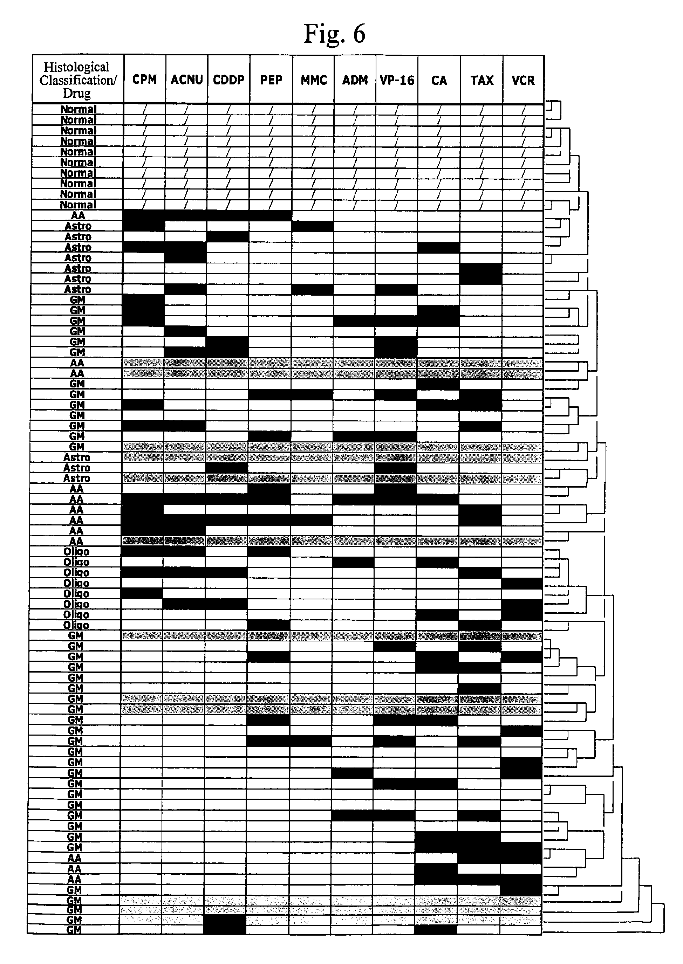

Figure 6 shows a diagram of hierarchical clustering applied on each of the cases based on the proteome profiling data determined for 75 glioblastoma samples and 10 normal brain samples as well as 8 samples of oligodendroglioma which is known to be comparatively high in drug sensitivity. The hierarchical grade was classified into 5 groups consisting of normal brain tissue (Normal), astrocytoma (Astro), undifferentiated astrocytoma (AA), multiform glioblastoma (GM) and oligodendroglioma (Oligo). Sensitivity to 10 representative drugs (CPM, ACNU, CDDP, PEP, ADM, VP-16, CA, TAX and VCR) for which dose response relationship was evaluated is shown in the right column.

### Detailed Description of the Invention

The inventors of this application have investigated proteome profiling patterns among plural samples of human brain tumor using two-dimensional electrophoresis and matrix-associated laser desorption/ionization time-of-flight (MALDI-TOF) mass spectrometry and identified 16 kinds of proteins (SEQ ID NOS: 1-16) as shown in Table 1.

**Table 1**

| No. | Name of Protein | Amino acid sequence | | cDNA sequence | |
|---|---|---|---|---|---|
| | | Swissprot | Sequence Listing | GenBank | Sequence Listing |
| 1 | Nucleolar GTP-binding protein | Q9BZE4 | 1 | NM_012341 | 17 |
| 2 | Glutamate dehydrogenase 1 | 000367 | 2 | X07674 | 18 |
| 3 | Phosphopyruvate hydratase | P06733 | 3 | BT007163 | 19 |
| 4 | cAMP response element-binding protein | P16220 | 4 | X55545 | 20 |
| 5 | Phosphoglycerate mutase 1 | P18669 | 5 | J04173 | 21 |
| 6 | Rab3A, member Ras oncogene family | P20336 | 6 | NM_002826 | 22 |
| 7 | Ras-related protein Ral-A | P11233 | 7 | X15014 | 23 |
| 8 | Paxillin | P49023 | 8 | U14588 | 24 |
| 9 | T complex protein 1, alpha subunit | P17987 | 9 | NM_030752 | 25 |
| 10 | Protein disulphide isomerase A3 | P30101 | 10 | NM_005313 | 26 |
| 11 | Eucaryotic translation initiation factor 4A(p37) | P04765 | 11 | NM_001416 | 27 |
| 12 | Activated C kinase receptor | P25388 | 12 | NM_006098 | 28 |
| 13 | Fibrillarin | P22087 | 13 | NM_001436 | 29 |
| 14 | Apolipoprotein A-1 | P02647 | 14 | NM_000039 | 30 |
| 15 | Prohibitin | P35232 | 15 | NM_000039 | 31 |
| 16 | Transforming protein RhoA | P06749 | 16 | AF498970 | 32 |

And the inventors have further confirmed that these 16 kinds of proteins express in either one or more tissues of highly malignant brain tumor, low malignant brain tumor, drug sensitive brain tumor and drug resistant brain tumor. That is, the inventors have found that the proteins Nos. 1, 4, 5, 6 and 7 in Table 1 express in highly malignant brain tumor tissue and can be markers of highly malignant brain tumor; the proteins Nos. 2 and 3 express in low malignant brain tumor tissue and can be markers of low malignant brain tumor; the proteins Nos. 5, 8, 11, 13, 14, 15 and 16 express in drug sensitive brain tumor tissue and can be markers of drug sensitive brain tumor; and the proteins Nos. 1, 2, 9, 10, 12 and 16 express in drug resistant brain tumor tissue and can be markers of drug resistant brain tumor.

As for the proteins No. 1-16 in Table 1, the following functions are known.
No .1: This is present in the nucleolus and has an amino acid sequence homologous to the other G protein groups and participates in intracellular signal transduction through G protein-coupled receptors present in the cell membrane.
No .2: It is known that various kinds of metabolic enzyme activities are generally enhanced in some cancer cells.
No .3: Isoenzyme (NSE) specifically present in nerve cells is known, and it is considered to be reduced with decrease of normal nerve cells.
No .4: This is a transcription factor, reportedly has important roles in the fields such as inflammatory reaction and neuranagenesis and participates in transcription of target genes at the final step of signal transduction system from tyrosine kinase type receptors, which play important roles in canceration of various kinds of cells.
No .5: This is an enzyme participating in the metabolism of phosphoglycerate generated in glycolysis, and availability as a disease severity marker in cerebral apoplexy and myocardial infarction and increase in activity in some cancers have been reported.
No .6: This belongs to Rab family among G proteins, and is observed to be expressed in brain and endocrine tissues. It is considered that this is important for the formation of secretory granules in synapses and the like. There are few reports that pointed out association with tumorigenesis.
No .7: This belongs to Ras family among G proteins, and it is considered to participate in various kinds of diseases including cancer.
No .8: This participates in cell adhesion and also participates in signal transduction through the action on cell surface receptors and cytoskeleton.
No .9: This is a molecular chaperon important for the structure formation of cytoplasm proteins such as actin and tubulin, and association with large bowel cancer and a hepatocellular carcinoma has been pointed out.
No 10: This is located in endoplasmic reticula and functions as a molecular chaperon for the protein synthesized there.
No 11: This binds to RNA, and is a protein which controls the initiation of translation.
No 12: This interacts with protein kinase C and participates in cell adhesion and movement through integrin. This also has cell proliferation inducing action in certain cancer cells.
No .13: This is a protein which is present in endoplasmic reticula and interacts with RNA and has chaperon function.
No 14: This participates in excretion of lipid from cells, and the reduction thereof can be a risk factor of ischemic heart disease along with HDL. In the meantime, lipid component is essential to cell membrane synthesis, and it is known that the level thereof decreases in fast proliferating cells.
No .15: This is a molecular chaperon localized in the membrane of mitochondria. Suppressive effect on cell proliferation is also known, and this is a protein having cancer suppressive effect.
No .16: This belongs to Rho family among G proteins, and it is known to participate in regulating cytoskeleton as well as in malignant transformation of cells. In late years, it is reported that the expression thereof decreases as malignancy grade of brain tumor elevates.

However, as for these 16 kinds of proteins, correlation with high/low malignancy and/or drug sensitivity/resistance of brain tumor has not been known at all.

The invention of this application is based on new marker proteins and polynucleotides encoding the same as described above.

Here, in the present invention, "protein" and "peptide" mean a molecule consisting of plural amino acid residues linked by an amide bond (peptide bond) with each other. "Polynucleotide" refers to a molecule having 100 or more linked phosphate esters of nucleosides (ATP, GTP, CTP, UTP; or dATP, dGTP, dCTP, dTTP) in which purine or pyrimidine binds to sugar through β-N-glycoside bond, and "oligonucleotide" refers to a molecule in which 2-99 such phosphate esters of nucleosides are linked.

In addition, as for the nucleotide and amino acid sequences shown in Table 1, addition, deletion, or substitution for other bases of one or more bases (in other words, one or more mutations), or addition, deletion, or substitution for other amino acids of one or more amino acids based on such mutations of bases (in other words, one or more mutations) are also included.

Furthermore, "highly malignant tumor" means a tumor which has high proliferation capability and invasive ability and becomes fatal at early stage. Therefore, "highly malignant tumor" and "the low malignant tumor" in the present invention can be specifically distinguished by the criteria that the case where the survival time is within one and half years due to high proliferation capability and invasive ability is judged as "high malignancy" while the case where the survival time is two or three or more years is judged as "low malignancy".

In addition, "drug sensitivity" means that there is an anti-cancer agent which can induce cell death in such a blood concentration as obtained by usual clinical dosage and "drug resistance" means that there is no such anti-cancer agents at the time point when the the present invention is completed. The drug sensitive brain tumor means brain tumor cells which will be annihilated by or the proliferation capability of which will be eliminated or reduced by, or the invasive ability of which will be eliminated or reduced by anti-cancer agents, for example, alkylating agents (4-hydroperoxy-cyclophosphamide (CPM) (1 µg/ml), 4-hydroperoxy-ifosfamide (IFOS) (1 µg/ml), melphalan (MPL) (0.5 µg/ml), carboquone (CQ) (0.1 µg/ml), nimustine (ACNU) (2 µg/ml), ranimustine (MCNU) (2 µg/ml), cisplatinum (CDDP) (0.5 µg/ml) and carboplatin (JM-8) (4 µg/ml)); antimetabolites (methotrexate (MTX) (3 µg/ml), 5-fluorouracil (5-FU) (10 µg/ml), thioinosine (6-MRP) (3 µg/ml) and cytosine arabinoside (CA) (4 µg/ml)); antibiotics (mitomycin C (MMC) (0.2 µg/ml), bleomycin (BLM) (1 µg/ml), peplomycin (PEP) (0.5 µg/ml), chromomycin A3 (TM) (0.01 µg/ml) and neocarsinostatin (NCS) (0.15 µg/ml)); topoisomerase inhibitor (actinomycin D(ACD) (0.01 µg/ml), aclarubicin (ACR) (0.6 µg/ml), adriamycin (ADM) (0.3 µg/ml), daunomycin (DM) (0.6 µg/ml), pirarubicin (THP) (0.3 µg/ml),epirubicin (4'-EPI) (0.4 µg/ml), mitoxantrone (MIT) (0.06 µg/ml), etoposide (VP-16) (3 µg/ml) and camptothecin (CPT-11) (3 µg/ml)); microtubule inhibitor (vincristine (VCR) (0.1 µg/ml), vinblastin (VLB) (0.1 µg/ml), vindesin (VDS) (0.1 µg/ml) and paclitaxel (TAX) (0.6 µg/ml)).

In addition, "diagnosis" in the present invention means a judgment whether a subject suffers from brain tumor, a judgment whether there is a risk that a subject will suffer from brain tumor in the future, a judgment on the effect of treatment and a judgment whether there is a risk that brain tumor will relapse after treatment. The diagnosis also includes determination of degree and risk of brain tumor.

Other terms and concept in the present invention will be prescribed in detail in the description on the embodiments of the invention and Examples. In addition, various techniques to be used to carry out the present invention can be conducted readily and with certainty by those skilled in the art based on known documents, etc. except for such techniques as specifically shown with references. For example, techniques of gene engineering and molecular biology can be performed by a method of described in or a method described in a reference cited in or a method substantially the same as or modified from the followng: J. Sambrook, E. F. Fritsch & T. Maniatis, "Molecular Cloning: A Laboratory Manual (2nd edition)", Cold Spring Harbor Laboratory Press, Cold Spring Harbor, New York (1989); D.M. Glover et al. ed., "DNA Cloning", 2nd ed., Vol. 1 to 4, (The Practical Approach Series), IRL Press, Oxford University Press (1995); Ausubel, F. M. et al., Current Protocols in Molecular Biology, John Wiley & Sons, New York, N.Y, 1,995; Japanese Biochemical Society ed., "Zoku Seikagaku Jikken Kouza 1, Idenshi Kenkyu-hou II"("Sequel Biochemical Experiment Series 1, Methods for Gene Study II"), Tokyo Kagaku Dojin (1986); Japanese Biochemical Society ed., "Shin Seikagaku Jikken Kouza 2, Kakusan III (Kumikae DNA Gijutsu)"("New Biochemical Experiment Series 2, Nucleic Acid III" (Recombinant DNA Technique)"), Tokyo Kagaku Dojin (1986); R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154(Recombinant DNA, Part E) & 155 (Recombinant DNA, Part F), Academic Press, New York (1987); J.H. Miller ed., "Methods in Enzymology", Vol. 204, Academic Press, New York (1991); R. Wu et al. ed., "Methods in Enzymology", Vol. 218, Academic Press, New York (1993); S. Weissman (ed.), "Methods in Enzymology", Vol. 303, Academic Press, New York (1999); J.C.Glorioso et al. (ed.) "Methods in Enzymology", Vol. 306, Academic Press, New York (1999), etc.

According to the invention of this application, tumor marker proteins to be used for simply and accurately judging high/low grade of malignancy of brain tumor and/or presence/absence of drug sensitivity are provided. Various molecular biological materials relating to these marker proteins are also provided and the grade of malignancy and/or drug sensitivity of brain tumor can be diagnosed precisely by using these, and thereby selection of an effective therapeutic method is enabled.

### Best Mode For Carrying Out the Invention

The brain tumor marker according to the invention (1) is each of proteins of Nos. 1-16 shown in Table 1. These proteins are characterized in that they have amino acid sequences represented by SEQ ID NOS: 1-16 respectively, each protein being expressed in at least one tissue of a highly malignant brain tumor, a low malignant brain tumor, a drug sensitive brain tumor and a drug resistant brain tumor.

These brain tumor markers are classified into groups of the following inventions (2) to (5).

The brain tumor markers according to the invention (2) are protein Nos. 1, 4, 5, 6 and 7 shown in Table 1 (SEQ ID NOS: 1,4, 5, 6 and 7, respectively), which are highly malignant tumor marker proteins expressed in a highly malignant brain tumor.

The brain tumor markers according to the invention (3) are protein Nos. 2 and 3 shown in Table 1 (SEQ ID NOS: 2 and 3, respectively), and they are low malignant brain tumor markers expressed in low malignant brain tumor tissues.

Furthermore, the brain tumor markers according to the invention (4) are protein Nos. 5, 8, 11, 13, 14, 15 and 16 shown in Table 1 (SEQ ID NOS: 5, 8, 11, 13, 14, 15 and 16, respectively), and they are drug sensitive brain tumor markers expressed in drug sensitive brain tumor tissues.

Furthermore, the brain tumor markers according to the invention (5) are protein Nos. 1, 2, 9, 10, 12 and 16 shown in Table 1 (SEQ ID NOS: 1, 2, 9, 10, 12 and 16, respectively), and they are drug resistant brain tumor markers expressed in drug resistant brain tumor tissues.

The marker proteins according to these inventions (1) to (5) can be used, for example, as antigens for producing antibodies according to the invention (6) using purified products thereof, respectively.

The purified product of each marker protein can be obtained by a method comprising isolating and purifying them from a human cell (for example, cell from brain tumor tissue), or can be obtained by a genetic engineering method using a polynucleotide (cDNA) consisting of a nucleotide sequence shown in each of SEQ ID NOS: 17-32. For example, RNA may be prepared by *in vitro* transcription from a recombination expression vector carrying each cDNA and may be expressed *in vitro* by performing *in vitro* translation using the cDNA as a template. They can be expressed in a transformed cell prepared by introducing a recombinant expression vector into a procaryotic cell such as E. coli or Bacillus subtilis or a eucaryotic cell such as yeast, insect cell or mammalian cell.

When the marker protein is expressed by *in vitro* translation, a recombination expression vector may be prepared by inserting a polynucleotide into a vector having an RNA polymerase promoter and adding this vector to an *in vitro* translation system such as rabbit reticular cell lysate or wheat germ extract including RNA polymerase corresponding to the promoter, and the protein of interest can be produced *in vitro.* Examples of RNA polymerase promoters include T7, T3 and SP6. Examples of vectors including such RNA polymerase promoter include pKA1, pCDM8, pT3/T7 18, pT7/3 19 and pBluescript II.

When the marker protein is expressed in microorganism such as E. coli, an expression vector, in which a vector having origin, promoter, ribosome-binding site, DNA cloning site, terminator, etc. and replicable in the microorganism is recombined with a polynucleotide, may be prepared, and the protein which the polynucleotide encodes can be expressed in the microorganism by transforming a host cell with this expression vector and culturing the obtained transformant, wherein it can be expressed as a protein fused with another protein. Examples of expression vectors for E. coli include pUC system, pBluescript II, pET expression system and pGEX expression system.

When the marker protein is expressed in an eucaryotic cell, a recombinant vector may be prepared by inserting a polynucleotide into an expression vector for eucaryotic cell having promoter, splicing region, poly (A) additional site and so on, and the protein of interest can be expressed in a transformed eucaryotic cell by introducing the recombinant vector into an eucaryotic cell. Examples of expression vectors include pKA1, pCDM8, pSVK3, pMSG, pSVL, pBK-CMV, pBK-RSV, EBV vector, pRS, pcDNA3, pMSG and pYES2. The protein can be also expressed as a fusion protein to which any of various tags such as His tag, FLAG tag, myc tag, HA tag, and GFP is added when pIND/V5-His, pFLAG-CMV-2, pEGFP-N1, pEGFP-Cl or the like is used as an expression vector. Generally, for eucaryotic cells, cultured mammalian cells such as monkey kidney cell COS7, Chinese hamster ovary cell CHO, budding yeast, fission yeast, silkworm cell, and Xenopus oocyte can be used, but any kind of eucaryotic cell is usable as long as it can express the marker proteins of the present invention. In order to introduce the expression vector into the eucaryotic cell, well-known methods such as electroporation, calcium phosphate method, liposome method, and DEAE dextran method can be used.

Conventional separation procedures may be used in combination for isolating and purifying the peptide of interest from the culture after expressing the marker protein in a procaryotic or eucaryotic cell. Examples of such methods include treatment with a denaturant or surfactant such as urea, ultrasonication, enzymatic digestion, salting out and solvent sedimentation method, dialysis, centrifugation, ultrafiltration, gel filtration, SDS-PAGE, isoelectric focusing, ion exchange chromatography, hydrophobic chromatography, affinity chromatography, and reverse phase chromatography.

The recombinant protein obtained by the above-mentioned method can be fused with any other protein in order to obtain a fusion protein. Examples thereof include fusion proteins with glutathione-S-transferase (GST) or green fluorescence protein (GFP). Furthermore, the protein expressed in the transformed cell may be subjected to various modifications in the cell after it was translated. Therefore, modified proteins are included in the scope of the protein of the present invention. Examples of such a post-translational modification include elimination of N-terminal methionine, N-terminal acetylation, sugar chain addition, limited proteolysis by intracellular protease, myristoylation, isoprenylation, and phosphorylation.

The antibody of invention (6) is a polyclonal antibody or a monoclonal antibody, and includes any of the whole antibody molecule and Fab, F(ab')₂ and Fv fragments thereof, which can bind to an epitope of each of the proteins having amino acid sequences of SEQ ID NOS: 1-16. Such an antibody can be obtained, for example, from the blood serum after immunizing an animal with the protein or a fragment thereof as an immunogen in case of polyclonal antibody. Alternatively, the antibody can be prepared by introducing an expression vector for eucaryotic cell as mentioned above into the muscle or skin of an animal by injection or by a gene gun and then collecting the blood serum. As the animal, mouse, rat, rabbit, goat, or chicken can be used.

The monoclonal antibody can be prepared following known methods for preparing a monoclonal antibody ("Monoclonal Antibody", Komei Hasemune and Hiroshi Terada, Hirokawa Shoten, 1990; "Monoclonal Antibody" James W. Goding, third edition, Academic Press, 1996).

The antibody of the present invention (6) includes an antibody labeled with a labeling substance. Enzyme, radioisotope or fluorochrome can be used as a labeling substance. There is not particular limitation on the enzyme as long as it satisfies conditions such as a large turnover number, stability in binding to the antibody, and specific staining of the substrate, and enzymes used for normal EIA include, for example, peroxidase, β-galactosidase, alkaline phosphatase, glucose oxidase, acetylcholine esterase, glucose-6-phosphate dehydrogenase, malate dehydrogenase, etc. Enzyme inhibitor, coenzyme or the like can be also used. Binding of such an enzyme to an antibody can be performed by well-known methods using a cross-linking agent such as maleimide compound. Known substances can be used depending on the kind of enzyme to be used. For example, when peroxidase is used as an enzyme, 3,3',5,5'- tetramethylbenzene can be used and when alkaline phosphatase is used as an enzyme, p-nitrophenol, etc. can be used. Examples of the radioisotope, include those conventionally used in RIA such as ¹²⁵I and ³H. For fluorochrome, those used in normal fluorescent antibody techniques such as fluorescein isothiocyanate (FITC) and tetramethylrhodamine isothiocyanate (TRITC) can be used.

The invention (7) is a brain tumor diagnostic kit including one or more kind of the above antibody and/or labeled antibody thereof. Such a diagnostic kit may include an antibody or a labeled antibody in the liquid phase or may have an antibody or a labeled antibody bound to the solid phase support. Furthermore, when the label of labeled antibody is an enzyme, this diagnostic kit may include the substrate therefor, for example, and when a solid phase support is included, a solution for washing to remove molecules not bound to the solid phase may be included. Various types of such kits are commercially available depending on the type of the analyte, and the diagnostic kit of the present invention can be composed of each element used for a conventionally known or used kit except that an antibody and/or a labeled antibody provided by the present invention is used.

Inventions (8) to (11) are polynucleotides (i.e., DNA fragments, or RNA fragments) encoding the marker proteins of the inventions (2) to (5), respectively. Specifically, they are genomic DNAs encoding each protein, mRNA transcripted from the genomic DNA, and cDNA synthesized from the mRNA. The cDNAs have nucleotide sequences of SEQ ID NOS: 17-32, respectively. They may be single-stranded or double-stranded. Furthermore, the sense and antisense strands of the genomic DNA, mRNA, or cDNA are also included. Still further, in the case of genomic DNA, expression controlling region (e.g., promoter, enhancer, or suppressor region) are included.

These polynucleotides can be readily obtained by a well-known method, respectively. For example, the cDNAs can be synthesized by using a well-known method (Mol. Cell Biol.2, 161 - 170, 1982; J. Gene 25, 263 - 269, 1983; Gene, 150, 243 - 250, 1994) and can be obtained by isolating each cDNA using probe DNAs prepared based on the respective nucleotide sequences from SEQ ID NOS: 1-16. The obtained cDNA can be amplified by, for example, usually performed gene amplification methods such as PCR (Polymerase Chain Reaction) method, NASBN (Nucleic acid sequence based amplification) method, TMA (Transcription-mediated amplification) method and SDA (Strand Displacement Amplification) method. Each cDNA can also be obtained in a required amount by RT-PCR method using primer sets as provided by the present invention and isolated mRNA from a human cell as a template.

The invention (12) is directed to DNA fragments consisting of each of partial contiguous sequences of SEQ ID NOS: 17-32. In addition, DNA fragments consisting of partial contiguous sequences of the sequences complementary to SEQ ID NOS 17-32 are also included in the DNA fragment. Preferably it is a DNA fragment of 6 to 75 nucleotides, more preferably 10 to 40 nucleotides, and most preferably 15 to 30 nucleotides.

This DNA fragment can be obtained, for example, by cleaving the above-mentioned polynucleotide (cDNA) with a suitable restriction enzyme. Alternatively, it can be synthesized *in vitro* by well-known chemosynthesis techniques described in Carruthers (1982) Cold Spring Harbor Symp. Quant. Biol.47:411-418; Adams (1983) J. Am. Chem. Soc. 105:661; Belousov (1997) Nucleic Acid Res. 25:3440-3444, Frenkel (1995) Free Radic. Biol. Med. 19:373-380; Blommers (1994) Biochemistry 33:7886-7896; Narang (1979) Meth.Enzymol. 68:90; Brown (1979) Meth. Enzymol. 68:109; Beaucage (1981) Tetra.Lett.22:1,859 and US Patent No. 4,458,066. This DNA fragment can be used as a probe by labeling with a labeling substance as in the above-mentioned polynucleotide. Labeling can be performed by radioisotope (RI) method or non-RI method, but it is preferable to use non-RI method. The non-RI method includes fluorescent labeling methods, biotin labeling methods, chemiluminescence methods and the like, but it is preferable to use fluorescent labeling methods. As for the fluorescent substances, those which can be bound to the basic part of the oligonucleotide can be suitably selected and used but cyanine coloring agent (for example, Cy3 or Cy5 of Cy Dye^{™} series), rhodamine 6G reagent, N-acetoxy-N²-acetylaminofluorene (AAF), AAIF (iodine derivative of AAF) and the like can be used.

Furthermore, DNA fragment of the invention (12) is characterized in that the fragment hybridizes under stringent condition with a polynucleotide of the above-described inventions (8) to (11). The stringent condition as used herein is a condition which enables the selective and detectable specific binding between the above-mentioned polynucleotide and DNA fragment probe. The stringent condition is defined with salt concentration, organic solvent (for example, formamide), temperature and other well-known conditions. In other words, the stringency increases as the salt concentration is reduced, the organic solvent concentration is increased or the hybridization temperature is elevated. For example, stringent salt concentration is usually about 750 mM or less NaCl and about 75 mM or less trisodium citrate, more preferably about 500 mM or less NaCl and about 50 mM or less trisodium citrate, most preferably about 250 mM or less NaCl and about 25 mM or less trisodium citrate. Stringent organic solvent concentration is about 35% or more and most preferably about 50% or more of formamide. The stringent temperature condition is about 30°C or more, more preferably about 37°C or more and most preferably about 42°C or more. The other conditions include hybridization time, concentration of detergent (for example, SDS), and presence/absence of carrier DNA, etc., and various stringencies can be set by combining these conditions.

According to one preferable embodiment, hybridization is performed under the condition of 750 mM NaCl, 75 mM trisodium citrate and 1% SDS at a temperature of 30°C. In a more preferable embodiment, hybridization is performed under the condition of 500 mM NaCl, 50 mM trisodium citrate, 1% SDS, 35% formamide and 100 µg/ml of denatured salmon sperm DNA at a temperature of 37°C. In the most preferable embodiment, hybridization is performed under the condition of 250 mM NaCl, 25 mM trisodium citrate, 1% SDS, 50% formamide and 200 µg/ml of denatured salmon sperm DNA at a temperature of 42°C. Washing condition after hybridization also influences the stringency. This washing condition is also defined with salt concentration and temperature, and the stringency of washing is increased by decreasing the salt concentration and elevating the temperature. For example, the salt condition which is stringent for washing is preferably about 30 mM or less NaCl and about 3 mM or less trisodium citrate, most preferably about 15 mM or less NaCl and about 1.5 mM or less trisodium citrate. The temperature condition which is stringent for washing is about 25°C or more, more preferably about 42°C or more and most preferably about 68°C or more. According to one preferable embodiment, washing is performed under the condition of 30 mM NaCl, 3 mM trisodium citrate and 0.1% SDS at a temperature of 25°C. In more preferable embodiment, washing is performed under the condition of 15 mM NaCl, 1.5 mM trisodium citrate, 0.1% SDS at a temperature of 42°C. In the most preferable embodiment, washing is performed under the condition of 15 mM NaCl, 1.5 mM trisodium citrate, 0.1% SDS at a temperature of 68°C.

The invention (13) is a DNA microarray comprising a polynucleotide(s) of the above-described inventions (8) to (11) and/or a DNA fragment(s) of the above-described invention (14).

Methods used for the preparation of microarray are methods of directly synthesizing an oligonucleotide on solid-phase carrier surface (on-chip synthesis), and methods of fixing an oligonucleotide or polynucleotide prepared beforehand on a solid phase carrier surface. The microarray to be used in the present invention can be prepared by either of these methods. The on-chip synthesis can be performed, for example, by a method of selectively synthesizing at a predetermined minute area on the matrix by combination of the use of a protection group which can be selectively removed by radiation of light, with the photolithography technology used in the production of semiconductor and the solid phase synthesis technology (masking technology: for example, Fodor, S.P.A. Science 251:767, 1991). On the other hand, when an oligonucleotide or polynucleotide prepared beforehand is fixed on solid-phase carrier surface, the oligonucleotide or a polynucleotide to which a functional group has been bonded is synthesized and spotted to covalently bond to the solid phase carrier surface which has been subjected to surface treatment (for example, Lamture, J.B. et al. Nucl.Acids Res. 22:2121-2125, 1994; Guo, Z. et al. Nucl.Acids Res. 22:5456-5465, 1994).

The oligonucleotide or polynucleotide is generally covalently bonded to the surface-treated solid phase carrier through a spacer or a cross linker. There is also a known method in which minute pieces of polyacrylamide gel are aligned on the glass surface to which synthetic oligonucleotides are covalently bonded (Yershov, G. et al. Proc. Natl. Acad. Sci. USA 94:4913, 1996). There is also another known method in which an array of microelectrodes is formed on a silica microarray and a permeable agarose layer containing streptavidin is set on the electrodes as a reactive site, biotinylated oligonucleotides are fixed on the site by positively charging this site, and the charge is controlled on the site, thereby enabling high-speed and precise hybridization (Sosnowski, R.G. et al. Proc. Natl. Acad. Sci. USA 94:1,119-1123, 1997).

The Invention (14) is a primer set for amplifying the nucleotide sequences of SEQ ID NOS: 17 to 32 by PCR.

These primer sets can be designed based on each of he nucleotide sequences of SEQ ID NOS: 17-32 and can be prepared through the synthesis and purification steps. As kept in mind upon the primer design, the following can be pointed out for example. The size (the number of bases) of a primer is 15-40 bases, desirably 15-30 bases in consideration of satisfying specific annealing between the primer and the template DNA. However, at least 30 bases are effective when LA (long accurate) PCR is performed. Complementary sequences between the both primers should be avoided so that a set of or a pair (2 strands) of primers consisting of sense (at 5'-end side) and antisense (at 3'-end side) strands may not anneal with each other, and a self-complementary sequence should be also avoided to prevent the formation of hairpin structure in the primers. Furthermore, GC content should be about 50% to secure stable binding to the template DNA, and GC-rich or AT-rich regions should not be unevenly distributed within the primers. Because the annealing temperature depends on Tm (melting temperature), primers having Tm values in the range of 55 to 65°C and similar to each other are to be selected in order to obtain highly specific PCR products. In addition, it is necessary to note that the final concentration of the primers used in PCR should be adjusted to about 0.1 to about 1 µM. Commercially available softwares for primer designing, for example, Oligo^{™} [product of National Bioscience Inc. (U.S.A.)], GENETYX [product of Software Development Co., Ltd. (Japan)] can be also used.

With materials provided by each of the above-described inventions, diagnosis of brain tumor, particularly judgment and diagnosis of the characteristics of the brain tumor (high/low grade of malignancy, presence/absence of drug sensitivity) are enabled.

The method of the inventions (15) to (18) is a method for diagnosing brain tumor using as an indicator the expression levels of the polynucleotides of the above-described inventions (8) to (11), respectively. Specifically, the invention (15) is a method for diagnosing highly malignant brain tumor using as an indicator the expression level of the polynucleotide of the invention (8); the invention (16) is a method for diagnosing low malignant brain tumor using as an indicator the expression level of the polynucleotide of the invention (9); the invention (17) is a method for diagnosing drug sensitive brain tumor using as an indicator the expression level of the polynucleotide of the invention (10); and the invention (18) is a method for diagnosing drug resistant brain tumor using as an indicator the expression level of the polynucleotide of the invention (11). The "polynucleotide" in this method is a genomic gene DNA encoding each of the above-mentioned tumor markers and means the one which is present in a tissue cell isolated from a subject. A "subject" is, for example, a patient who has received a medical examination and suspected to be with brain tumor by MRI inspection or the like, and the "biological sample" is an excised tissue from the brain, blood or the like of the patient. As a specific criteria, the level of polynucleotide expression of a subject relative to that of a healthy person is higher by 10% or more, preferably 30% or more, more preferably 70% or more, and most preferably 100% or more.

The expression level of the polynucleotide can be known by measuring transcription product (mRNA, protein) of the polynucleotide. For example, assay of transcription product is possible by in situ hybridization, Northern blotting, dot blotting, RNase protection assay, RT-PCR, Real-Time PCR (for example, Journal of Molecular Endocrinology, 25, 169-193, 2000 and references cited there), DNA microarray analysis method (for example, Mark Shena, ed., "Microarray Biochip Technology", Eaton Publishing, 2000), etc.

For example, the level of mRNA transcripted from a polynucleotide can be measured by northern blotting assay using a probe obtained by labeling the DNA fragment of the above-described invention (12), as an intensity of the label signal.

The level of the polynucleotide expression can be measured simply and extensively by using DNA microarray of the above-described invention (13). Specifically, cDNA may be synthesized, for example, using mRNA isolated from a cell of the subject as a template, and may be amplified by PCR. Labeled dNTP may be incorporated to form a labeled cDNA. The labeled cDNA is contacted with the macroarray, and the label signal is detected as an indicator of cDNA hybridized with a capture probe (oligonucleotide or polynucleotide) on the microarray. Hybridization can be carried out by dispensing an aqueous liquid of labeled cDNA to a 96-well or 384-well plastic plate for spotting onto the microarray. The spotted amount is around 1 to 100 n1. The hybridization is preferably performd in the range of room temperature to 70°C for 6 to 20 hours. It is preferable to wash with a mixture of detergent and buffer after the hybridization is completed. Example of the buffer used are citrate buffer, phosphate buffer, borate buffer, Tris buffer, Good's buffer, etc., preferably citrate buffer.

Alternatively, the transcription product mRNA of the polynucleotide can be also measured by the RT-PCR method using a primer set of the invention (15). Further, the level of the polynucleotide mRNA can be quantified by using the quantitative RT-PCR method.

In addition, when the protein (or tumor marker) is measured as a transcription product of the polynucleotide, the measurement can be performed using the antibody of the above-described invention (6) or the diagnostic kit of the above-described invention (7). Such methods using an antibody include, for example, immunostaining, such as tissue staining or cell staining, immunoelectron microscopy, and immunoassay such as competitive immunoassay or non-competitive immunoassay. Alternatively, radioimmunoassay (RIA), fluoroimmunoassay (FIA), luminescent immunoassay (LIA), enzyme immunoassay (EIA), ELISA and the like can also be used. B-F separation may or may not be performed before measurement. Preferable methods are RIA, EIA, FIA, and LIA, or sandwich assay. The sandwich assay may be simultaneous sandwich type assay, forward sandwich type assay, reverse sandwich type assay, or the like.

One aspect of such a diagnosis method using an antibody is a method of detecting the binding between the antibody and the brain tumor marker protein in a liquid phase system. For example, the labeled antibody of the invention (6) are contacted with a biological sample to bind the labeled antibody to the marker protein, and their conjugate is separated. Separation can be performed by methods of separating the conjugate of marker protein and labeled antibody, for example conventional separation means such as chromatography method, solid phase method, etc. Known conventional Western blotting method can also be used. When enzyme is used as a label, the label signal is determined by adding a substrate which is decomposed by action of the enzyme to develop a color, determining the enzymatic activity by optically measuring a decomposed amount of the substrate and converting it into the amount of bound antibody, and calculating an amount of the antibody through comparison with standard values. When radioisotope is used, the amount of the radiation emitted by the radioisotope is measured with a scintillation counter or the like. In addition, when fluorochrome is used, the amount of fluorescence can be measured with a measuring apparatus combined with a fluorescence microscope.

Another diagnostic method in a liquid phase system comprises contacting the antibody (as a primary antibody) of the invention (6) with a biological sample to bind the primary antibody to HRF protein, thereby forming a conjugate; binding a labeled antibody (as a secondary antibody) to the conjugate; and detecting the label signal from the conjugate of these three entities. Alternatively, non-labeled secondary antibody may be bound to the antibody and antigen peptide conjugate at first and subsequently a labeling substance may be bound to the secondary antibody in order to further enhance the signal. The binding of the labeling substance to the secondary antibody can be performed, for example, by biotinylating a secondary antibody while avidinating the labeling substance. Alternatively, third antibody recognizing a part of the region of the secondary antibody (for example, Fc region) may be labeled in order to bind it to the secondary antibody. Monoclonal antibodies can be used for both of the primary antibody and secondary antibody, or either one of the primary antibody and secondary antibody may be a polyclonal antibody. The separation of the conjugate from the liquid phase and the detection of the signal can be performed as described above.

Another aspect using an antibody is a method of testing binding of the antibody to the marker protein in a solid-phase system. Any method using solid-phase system is preferable in view of detection of an extremely small amount of the marker protein or in view of simplicity and easiness of the operation. In other words, in the solid-phase system, the antibody of the invention (6) is immobilized on a resin plate, membrane or the like, a marker protein is bound to this immobilized antibody, non-bound protein is washed out, after that, a labeled antibody is bound to the antibody and marker protein conjugate left on the plate, thereby detecting a signal from this labeled antibody. This method is a so-called "sandwich method," and when enzyme is used as a marker, this method is widely used as "ELISA" (enzyme linked immunosorbent assay). As for the two kinds of antibodies, monoclonal antibodies may be used for the both of them, or either one of them may be a polyclonal antibody.

The method of the invention (19) is a diagnosis method to judge which of the following brain tumors:
(a) highly malignant and drug sensitive;
(b) highly malignant and drug resistant;
(c) low malignant and drug sensitive; and
(d) low malignant and drug resistant
is the brain tumor of a subject by combining the diagnosis methods of the above-described inventions (15) to (18).

This method is extremely useful in the selection of therapeutic methods and so on because the grade of malignancy and the presence/absence of the drug sensitivity can be diagnosed at the same time. For example, when the tumor is judged as a highly malignant but drug sensitive, anti-cancer agent therapy having sensitivity can be selected. When the brain tumor is judged as a highly malignant and drug resistant, appropriate therapy such as radiotherapy or removal of a cancerous part can be selected.

The invention of this application will be described in more detail and more specifically by Examples but the invention is not limited by the following Examples.

### Examples

### Example 1

### Protein markers associated with the malignancy of brain tumor were identified.

### (1)Materials and methods

### (1-1) Tissue sample

75 human glioma tissues and noncancerous brain tissues surgically removed were used. Upon use of the tissues, an informed consent document was obtained from a patient or a guardian beforehand. 52 samples were diagnosed as multiform glioblastoma (GM, grade IV), 13 samples as undifferentiated astrocytoma (AA, grade III) and 10 samples as astrocytoma (grade II) according to the WHO standard. 10 normal brain samples were obtained from patients who received excision of off-axis brain tumor or an epilepsy operation. The excised tumors or the normal brain samples were divided into two parts immediately after they were surgically excised. One part of them was fixed in 10% formaldehyde and embedded in paraffin for histopathological diagnosis. The other part was snap-frozen in liquid nitrogen and stored at -80°C for extracting protein. The protocol of this experimental research was approved by Institutional Review Board aimed at analyzing a proteome profiling pattern in glioma.

### (1-2) Preparation of sample for two-dimensional gel

The frozen samples were mechanically homogenized in a lysis buffer containing 30 mM Tris-HCl (pH 7.5), 150 mM NaCl, 1% Triton X-100, 10% glycerol and a protease inhibitor cocktail to obtain a protein lysis liquid. Bradford assay using BioRad Protein Assay (Japan BioRad Company, Tokyo) was carried and the concentration of the total proteins were determined and standardized to 1 µg/µl for all samples. Each protein sample (100 µg) was freeze-dried and a sample buffer containing 7M urea, 2M thiourea, 4%(w/v) CHAPS and 1%(w/v) DTT was added thereto.

### (1-3) Two-dimensional gel electrophoresis

Isoelectric focusing (IEF) for the first dimension separation of proteins was carried out using Multiphore II electrophoresis system (Amersham Biosciences, New Jersey, USA) and Investigator 5,000V two-dimension power supply (Genomic Solutions, Inc., Ann Arbor, Michigan, USA) following the instructions of the manufacturers. An Immobiline Dry IPG strip (7cm, pH 3 to 10 non-linear IPG strip) produced by Amersham Biosciences company was swollen in with a mixed solution of sample/swelling buffer overnight. The strip was subjected to electrophoresis using a ramping IPG strip (200 to 5000V) electrophoresis algorithm attaining the final electrophoresis voltage of 3,500V and 3700Vh. Following IEF, the gel strip was equilibrated in an SDS equilibration buffer and subjected to electrophoresis in a vertical SDS-PAGE Slab gel containing 12.5% acrylamide. Some sample were subjected to electrophoresis with molecular weight (Mr) and isoelectric point (pI) calibration markers for calculating the molecular weight and isoelectric point of protein spots at image analysis. Iinmeadiately after the two-dimensional SDS-PAGE, the gel was stained with Silver Quest Ag Staining Kit (Invitrogen Co., Ltd., Tokyo) compatible with mass spectrometry.

### (1-4) Image analysis of two-dimensional gel

The Ag stains on the gel were digitized using a flatbed image scanner with a resolution of 300 dpi. Location and determination of the intensity of protein spots and matching of the spot between the paired gels were performed using Phoretix 2D Advanced software (Ver 5.01, Nonlinear Dynamic, Ltd., New Castle, U.K.). Spot matching was automatically carried out after manually identifying the common anchor spots. Matching was performed for gel images obtained from 10 normal brain tissue samples and a composite reference image including most of the possible spots was prepared. The gel test in duplicate for 10 normal brain samples was performed and reproducibility of the experiment was confirmed. Only the spots which were present/completely absent between the tumor and control gels were assumed to be altered spots. This strict standard was used in this research so that possibility of the later validation would be the highest throughout the de-regulated proteins.

### (1-5) MALDI-TOF mass spectrometry

Protein spots were excised from the gel and treated with ZipTip C18 (Millipore Co., Ltd., USA). The digestion in the gel was carried out in an enzyme solution containing 50 mM NH₄HCO₃, 5mM CaCl₂ and 12.5 ng/l trypsin. An aliquot of the purified sample was spotted on the matrix crystal of α-cyano-4-hydroxylcinnamic acid on the stainless steel target and air-dried. The determination of mass was carried out by AXIMA-CFR mass spectrometer (Shimadzu Corporation, Kyoto, Japan) equipped with a nitrogen laser (λ =337 mm) and delayed extraction. The laser desorbed cations were analyzed after having accelerated at 20kV in linear mode. The outer calibration was carried out with a normal oligonucleotide mixture. The proteins were identified by PMF method using Mascot Search (Matrix Science, Ltd., London, U.K.) on the Web.

### (1-6) Statistic analysis

Hierarchical cluster analysis was performed on the tumor and normal tissue samples. Identification analysis was carried out to find out proteins which had an influence on the histologic grade significantly. All the statistical analysis was carried out with SPSS for Windows (registered trademark) software (SPSS, Inc., Chicago, Illinois, USA).

### (2) Results

### (2-1) Proteome profiling pattern in normal brain tissue and glioma

The protein expression was comprehensively analyzed in 75 glioma samples using two-dimensional electrophoresis technique of immobilized pH gradient method (Figure 1). About 400 protein spots in each gel were identified using conservative spot selection software algorithm based on the constant spot intensity. Many protein spots were differently expressed for each of the samples (Figure 2). The composite reference gel including 10 normal brain tissues included 631 protein spots in all. Matching of 64 ± 6.2% was observed in average for astrocytoma by comparing the reference gel and gels from each sample of glioma, and matching of 47 ± 4.9% (p <0.005) was observed in average for GM. 181 protein spots (28.7%) among the spots in the reference gel did not match with any spot in the other gel derived from glioma tissue. These proteins may be specifically translated and may function in normal brain. The rest of the proteins which did not match appeared only in one gel and therefore it is considered that tumor de-regulated proteins are extremely sample-specific and that they are proteolysis fragments and proteins modified after translation. There were also some protein spots which were possible to be identified by positive gel matching with protein expression patterns from some existing two-dimensional gel databases.

### (2-2) Cluster analysis of proteome profiling pattern of human glioma

Hierarchical clustering of 75 tumor samples and 10 normal brain samples was performed based on the expression of the protein spots and a proteome profiling pattern in human glioma associated with various biochemical characteristics were identified. A phylogenetic tree on the sample axis is shown in Figure 3. Samples obtained from normal brain tissues were completely classified into a single tissue specific branch, and appropriateness of the proteome analysis and clinical importance were demonstrated. Seven samples of astrocytoma gathered in the region near to that of the normal brain tissue, and the patient showed a good clinical course without recurrence. On the other hand, two people of the three remaining patients having astrocytoma and gathering away from the normal brain tissue had recurrence after the operation in 4 years or less. Most of GM having comparatively long survival time over two years were classified into a region near to that of low grade astrocytoma (Figure 3).

### (2-3) Identification of proteins associated with progress of malignancy in glioma

Identification analysis was carried out based on expression of protein spots in order to identify the proteins correlating to the histologic grade. The selected protein spots were excised and analyzed by MALDI-TOF MS assay (Figure 4). 25 known proteins and 12 unknown proteins were extracted by the analysis. The 25 known proteins above were classified into signal transduction associated proteins, molecular chaperon, transcription and translation regulators, cell cycle mediated protein, extracellular matrix associated protein and cell adhesion molecule based on the known or supposed biological properties. 8 signal transduction proteins were upregulated in the high grade tumors, and 5 of these 8 proteins were classified into low molecular weight G protein superfamily. There have been no reports showing that RalA, Rab3B and nucleus GTP-binding protein, which have been recognized as a new G protein family in late years among the proteins identified here, were related to malignant transformation of glioma. The upregulated Rho family (RhoA and Racl) may cause inhibition of transfer of actin filament mediated tumor cells and apoptosis, partially through a group of proteins, ezrin/radixin/moesin (ERM) group. MAPK and that PKC have been also identified as proteins upregulated in high grade tumors. Annexin family, classified into phospholipid bond and Ca bond membrane associated enzymes, was frequently upregulated in high grade glioma. 4 molecular chaperons have shown correlation with histologic grade. 3 of these 4 proteins were significantly upregulated in low grade tumors. In addition, upregulation or downregulation of three metabolism enzyme, phosphoglycerate mutase, glutamate dehydrogenase and phosphopyruvate hydratase have shown significant correlation with histologic grade.

### Example 2

Protein markers associated with drug sensitivity and drug resistance of brain tumor were identified.

A drug sensitivity test using flow cytometry followed a method described in a reference (Iwadate,Y., et al., Int J Cancer 69:236-240, 1996; Iwadate, Y., et al., Int J Mol Med 10: 187-192, 2002). That is, a tumor cell suspension was incubated together with 30 kinds of different anti-cancer agents for 8 hours and subsequently further cultured in a fresh culture medium which did not include a drug. These drugs were classified into five following categories by pharmacological mechanism.

### Alkylating agents:

4-hydroperoxy-cyclophosphamide (CPM)(1 µg/ml), 4-hydroperoxy-ifosfamide (IFOS) (1 µg/ml), melphalan (MPL) (0.5 µg/ml), carboquone (CQ) (0.1 µg/ml), nimustine (ACNU) (2 µg/ml), ranimustine (MCNU) (2 µg/ml), cisplatinum (CDDP) (0.5 µg/ml) and carboplatin (JM-8) (4 µg/ml)

### Antimetabolites:

Methotrexate (MTX) (3 µg/ml), 5-fluoro uracil (5-FU) (10 µg/ml), thioinosine (6-MRP) (3µg/ml) and cytosine arabinoside (CA) (4 µg/ml)

### Antibiotics:

Mitomycin C (MMC) (0.2 µg/ml), bleomycin (BLM) (1 µg/ml), peplomycin (PEP) (0.5 µg/ml), chromomycin A3 (TM) (0.01 µg/ml) and neocarsinostatin (NCS) (0.15 µg/ml)

### Topoisomerase inhibitors:

Actinomycin D (ACD) (0.01 µg/ml), aclarubicin (ACR) (0.6 µg/ml), adriamycin (ADM) (0.3 µg/ml), daunomycin (DM) (0.6 µg/ml), pirarubicin (THP) (0.3 µg/ml), epirubicin (4'-EPI) (0.4 µg/ml), mitoxantrone (MIT) (0.06 µg/ml), etoposide (VP-16) (3 µg/ml) and camptothecine (CPT-11) (3 µg/ml)

### Microtubule inhibitors:

Vincristine (VCR) (0.1 µg/ml), vinblastin (VLB) (0.1 µg/ml), vindesin (VDS) (0.1 µg/ml) and paclitaxel (TAX) (0.6 µg/ml)

The *in vitro* concentrations of these drugs were set to about 1/10 of the highest blood concentration when administered at a dose clinically recommended, and differences in the reaction by dose were evaluated for 10 selected drugs (CPM, ACNU, CDDP, PEP, ADM, VP-16, CA, TAX, VCR). The cultured samples of these were mixed in a phosphate buffered saline (pH 7.2)/0.1% Triton X-100/0.1 mg/ml RNase/0.01% sodium azide for 15 minutes and subsequently mixed with 50 g/ml of propidium iodide (Sigma,St.Louis, MO) for 5 minutes. The isolated nuclei were analyzed with flow cytometer (FACScan:Becton Dickinson, Mountain view, CA). The efficacy of a drug was judged by shifting the nucleus of apoptosis to a low diploid (sub-G1) region and comparing reverse decrease of the peak of the thus incorporated diploid with decrease of untreated control cells. Cells were fixed with 70% ethanol and subjected to Giemsa's staining on a slide glass to morphologically confirm the drug induced cell death (Iwadate, Y., et al., Int J Mol Med 10:187-192, 2002). Decomposition and condensation of chromatin were judged as a marker of apoptosis and quantitatively evaluated by Fisher's exact test. This morphologic inspection revealed that the average ratio of tumor cells in the preparation unalterably exceeded 95%. The integrity of DNA evaluated by FCM correlated well to the morphologic change by apoptosis. In order to avoid false-negative results, when either one of the assays was judged to be positive, the drug was eventually determined to be effective *in vitro*.

Correlation between the degree of drug sensitivity obtained by the above-mentioned method and proteome profiling patterns was examined. The drug sensitivity showed variability case by case (Figure 5). The drug sensitivity showed characteristic distribution (Figure 6) in the phylogenetic tree in the sample axis obtained in the same way as in Example 1 (2). That is, an alkylating agent has a high efficacy for the tumor which gathered to the neighborhood to the normal brain tissue, but it was not effective at all for the tumor which gathered in a remote space from the normal brain tissue. A tumor having sensitivity to ACNU, one of the alkylating agent, was generally resistant to VCR, one of the microtubule inhibitor. Most of the tumors resistant to all the drugs gathered in a remote space from the normal brain tissue.

The tumors resistant to all the drugs were considered to be tumors which lost a cell death mechanism by apoptosis, and therefore, identification analysis was conducted based on presence/absence of effective drugs as in the same way as in Example 1 (2). As a result, 30 known proteins and 12 unknown proteins were extracted.

### Industrial Applicability

As described in detail heretofor, the invention of this application enables to precisely diagnose the grade of malignancy and/or drug sensitivity of brain tumor, and enables to select more effective therapies.

## Claims

1. A brain tumor marker, **characterized in that** the brain tumor marker is at least one protein expressed in a brain tumor tissue and selected from the group of proteins having the amino acid sequences represented by SEQ ID NOS: 1 to 16, respectively, wherein the protein is expressed in at least one tissue selected from the group of the tissues of a highly malignant brain tumor, a low malignant brain tumor, a drug-sensitive brain tumor, and a drug-resistant brain tumor.

2. A highly malignant brain tumor marker, wherein the brain tumor marker is at least one protein selected from the group of proteins expressed in a highly malignant brain tumor tissue, wherein the protein has the amino acid sequence represented by SEQ ID NO: 1, 4, 5, 6 or 7.

3. A low malignant brain tumor marker, wherein the brain tumor marker is at least one protein selected from the group of proteins expressed in a low malignant brain tumor tissue, wherein the protein has the amino acid sequence represented by SEQ ID NO: 2 or 3.

4. A drug-sensitive brain tumor marker, wherein the brain tumor marker is at least one protein selected from the group of proteins expressed in a drug-sensitive brain tumor tissue, wherein the protein has the amino acid sequence represented by SEQ ID NO: 5, 8, 11, 13, 14, 15 or 16.

5. A drug-resistant brain tumor marker, wherein the brain tumor marker is at least one protein selected from the group of proteins expressed in a drug-resistant brain tumor tissue, wherein the protein has the amino acid sequence represented by SEQ ID NO: 1, 2, 9, 10, 12 or 16.

6. An antibody which binds to a protein selected from the group of proteins having the amino acid sequences represented by SEQ ID NOS: 1 to 16, respectively.

7. A diagnostic kit, **characterized in that** the diagnostic kit comprises at least one of the antibody according to claim 6 and/or a labeled antibody derived therefrom.

8. A polynucleotide encoding the highly malignant brain tumor marker according to claim 2.

9. A polynucleotide encoding the low malignant brain tumor marker according to claim 3.

10. A polynucleotide encoding the drug-sensitive brain tumor marker according to claim 4.

11. A polynucleotide encoding the drug-resistant brain tumor marker according to claim 5.

12. A DNA fragment consisting of a partial contiguous sequence of any one of SEQ ID NOS: 17 to 32.

13. A DNA microarray comprising the polynucleotide according to any one of claims 8 to 11 and/or the DNA fragment according to claim 12.

14. A set of primers for amplifying by PCR any one of the nucleotide sequences of SEQ ID NOS: 17 to 32.

15. A method of diagnosing a highly malignant brain tumor, **characterized in that** a biological sample from a subject is assayed for the expression level of the polynucleotide according to claim 8, wherein the subject is judged to be a patient with a highly malignant brain tumor if the subject has a higher expression level of at least one said polynucleotide than a healthy person.

16. A method of diagnosing a low malignant brain tumor, **characterized in that** a biological sample from a subject is assayed for the expression level of the polynucleotide according to claim 9, wherein the subject is judged to be a patient with a low malignant brain tumor if the subject has a higher expression level of at least one said polynucleotide than a healthy person.

17. A method of diagnosing a drug-sensitive brain tumor, **characterized in that** a biological sample from a subject is assayed for the expression level of the polynucleotide according to claim 10, wherein the subject is judged to be a patient with a drug-sensitive brain tumor if the subject has a higher expression level of at least one said polynucleotide than a healthy person.

18. A method of diagnosing a drug-resistant brain tumor, **characterized in that** a biological sample from a subject is assayed for the expression level of the polynucleotide according to claim 11, wherein the subject is judged to be a patient with a drug-resistant brain tumor if the subject has a higher existence level of at least one said polynucleotide than a healthy person.

19. A method of diagnosing a brain tumor, **characterized in that** said method combines the respective methods according to any one of claims 15 to 18, wherein a brain tumor which a subject suffers from is judged to be one selected from the group of:
(a) highly malignant and drug-sensitive;
(b) highly malignant and drug-resistant;
(c) low malignant and drug-sensitive; and
(d) low malignant and drug-resistant.


SEQUENCE LISTING

<110> Japan Science and Technology Agency

<120> Brain tumor marker and method of diagnosing brain tumor

<130> PH-2104-PCT

<140> PCT/JP2004/006345
<141> 2004-04-30

<150> JP 2003-309356
<151> 2003-09-01

<160> 32

<170> PatentIn version 3.1

<210> 1
<211> 633
<212> PRT
<213> Homo sapiens

<300>
<308> Swissprot/Q9BZE4
<309> 2002-06-15
<313> (1)..(633)

<400> 1

Met Ala His Tyr Asn Phe Lys Lys Ile Thr Val Val Pro Ser Ala Lys
1               5                   10                  15

Asp Phe Ile Asp Leu Thr Leu Ser Lys Thr Gln Arg Lys Thr Pro Thr
            20                  25                  30

Val Ile His Lys His Tyr Gln Ile His Arg Ile Arg His Phe Tyr Met
        35                  40                  45

Arg Lys Val Lys Phe Thr Gln Gln Asn Tyr His Asp Arg Leu Ser Gln
    50                  55                  60

Ile Leu Thr Asp Phe Pro Lys Leu Asp Asp Ile His Pro Phe Tyr Ala
65                  70                  75                  80

Asp Leu Met Asn Ile Leu Tyr Asp Lys Asp His Tyr Lys Leu Ala Leu
                85                  90                  95

Gly Gln Ile Asn Ile Ala Lys Asn Leu Val Asp Asn Val Ala Lys Asp
            100                 105                 110

Tyr Val Arg Leu Met Lys Tyr Gly Asp Ser Leu Tyr Arg Cys Lys Gln
        115                 120                 125

Leu Lys Arg Ala Ala Leu Gly Arg Met Cys Thr Val Ile Lys Arg Gln
    130                 135                 140

Lys Gln Ser Leu Glu Tyr Leu Glu Gln Val Arg Gln His Leu Ser Arg
145                 150                 155                 160

Leu Pro Thr Ile Asp Pro Asn Thr Arg Thr Leu Leu Leu Cys Gly Tyr
                165                 170                 175
Pro Asn Val Gly Lys Ser Ser Phe Ile Asn Lys Val Thr Arg Ala Asp
            180                 185                 190
Val Asp Val Gln Pro Tyr Ala Phe Thr Thr Lys Ser Leu Phe Val Gly
        195                 200                 205
His Met Asp Tyr Lys Tyr Leu Arg Trp Gln Val Val Asp Thr Pro Gly
    210                 215                 220
Ile Leu Asp His Pro Leu Glu Asp Arg Asn Thr Ile Glu Met Gln Ala
225                 230                 235                 240
Ile Thr Ala Leu Ala His Leu Arg Ala Ala Val Leu Tyr Val Met Asp
                245                 250                 255
Leu Ser Glu Gln Cys Gly His Gly Leu Arg Glu Gln Leu Glu Leu Phe
            260                 265                 270
Gln Asn Ile Arg Pro Leu Phe Ile Asn Lys Pro Leu Ile Val Val Ala
        275                 280                 285
Asn Lys Cys Asp Val Lys Arg Ile Ala Glu Leu Ser Glu Asp Asp Gln
    290                 295                 300
Lys Ile Phe Thr Asp Leu Gln Ser Glu Gly Phe Pro Val Ile Glu Thr

305                 310                 315                 320

Ser Thr Leu Thr Glu Glu Gly Val Ile Lys Val Lys Thr Glu Ala Cys

                325                 330                 335

Asp Arg Leu Leu Ala His Arg Val Glu Thr Lys Met Lys Gly Asn Lys

            340                 345                 350

Val Asn Glu Val Leu Asn Arg Leu His Leu Ala Ile Pro Thr Arg Arg

        355                 360                 365

Asp Asp Lys Glu Arg Pro Pro Phe Ile Pro Glu Gly Val Val Ala Arg

    370                 375                 380

Arg Lys Arg Met Glu Thr Glu Glu Ser Arg Lys Lys Arg Glu Arg Asp

385                 390                 395                 400

Leu Glu Leu Glu Met Gly Asp Asp Tyr Ile Leu Asp Leu Gln Lys Tyr

                405                 410                 415

Trp Asp Leu Met Asn Leu Ser Glu Lys His Asp Lys Ile Pro Glu Ile

            420                 425                 430

Trp Glu Gly His Asn Ile Ala Asp Tyr Ile Asp Pro Ala Ile Met Lys

        435                 440                 445

Lys Leu Glu Glu Leu Glu Lys Glu Glu Glu Leu Arg Thr Ala Ala Gly

    450                 455                 460

Glu Tyr Asp Ser Val Ser Glu Ser Glu Asp Glu Glu Met Leu Glu Ile
465                 470                 475                 480

Arg Gln Leu Ala Lys Gln Ile Arg Glu Lys Lys Lys Leu Lys Ile Leu
                485                 490                 495

Glu Ser Lys Glu Lys Asn Thr Gln Gly Pro Arg Met Pro Arg Thr Ala
            500                 505                 510

Lys Lys Val Gln Arg Thr Val Leu Glu Lys Glu Met Arg Ser Leu Gly
        515                 520                 525

Val Asp Met Asp Asp Lys Asp Asp Ala His Tyr Ala Val Gln Ala Arg
    530                 535                 540

Arg Ser Arg Ser Ile Thr Arg Lys Arg Lys Arg Glu Asp Ser Ala Pro
545                 550                 555                 560

Pro Ser Ser Val Ala Arg Ser Gly Ser Cys Ser Arg Thr Pro Arg Asp
                565                 570                 575

Val Ser Gly Leu Arg Asp Val Lys Met Val Lys Lys Ala Lys Thr Met
            580                 585                 590

Lys Asn Ala Gln Lys Lys Met Asn Arg Leu Gly Lys Lys Gly Glu Ala
        595                 600                 605

Asp Arg His Val Phe Asp Met Lys Pro Lys His Leu Leu Ser Gly Lys
    610                 615                 620

Arg Lys Ala Gly Lys Lys Asp Arg Arg
625                 630

<210>  2
<211>  558
<212>  PRT
<213>  Homo sapiens

<300>
<308>  Swissprot/O00367
<309>  2003-09-15
<313>  (1)..(558)

<400>  2

<210>  3

<211>  434

<212>  PRT

<213>  Homo sapiens

<300>

<308>  Swiisprot/P06733

<309>  2003-09-15

<313>  (1)..(434)

<400>  3

Met Ser Ile Leu Lys Ile His Ala Arg Glu Ile Phe Asp Ser Arg Gly

1                   5                   10                  15

Asn Pro Thr Val Glu Val Asp Leu Phe Thr Ser Lys Gly Leu Phe Arg

            20                  25                  30

Ala Ala Val Pro Ser Gly Ala Ser Thr Gly Ile Tyr Glu Ala Leu Glu

        35                  40                  45

Leu Arg Asp Asn Asp Lys Thr Arg Tyr Met Gly Lys Gly Val Ser Lys

    50                  55                  60

Ala Val Glu His Ile Asn Lys Thr Ile Ala Pro Ala Leu Val Ser Lys
65                  70                  75                  80

Lys Leu Asn Val Thr Glu Gln Glu Lys Ile Asp Lys Leu Met Ile Glu
                85                  90                  95

Met Asp Gly Thr Glu Asn Lys Ser Lys Phe Gly Ala Asn Ala Ile Leu
            100                 105                 110

Gly Val Ser Leu Ala Val Cys Lys Ala Gly Ala Val Glu Lys Gly Val
        115                 120                 125

Pro Leu Tyr Arg His Ile Ala Asp Leu Ala Gly Asn Ser Glu Val Ile
    130                 135                 140

Leu Pro Val Pro Ala Phe Asn Val Ile Asn Gly Gly Ser His Ala Gly
145                 150                 155                 160

Asn Lys Leu Ala Met Gln Glu Phe Met Ile Leu Pro Val Gly Ala Ala
                165                 170                 175

Asn Phe Arg Glu Ala Met Arg Ile Gly Ala Glu Val Tyr His Asn Leu
            180                 185                 190

Lys Asn Val Ile Lys Glu Lys Tyr Gly Lys Asp Ala Thr Asn Val Gly
        195                 200                 205

Asp Glu Gly Gly Phe Ala Pro Asn Ile Leu Glu Asn Lys Glu Gly Leu
    210                 215                 220

Glu Leu Leu Lys Thr Ala Ile Gly Lys Ala Gly Tyr Thr Asp Lys Val
225                 230                 235                 240

Val Ile Gly Met Asp Val Ala Ala Ser Glu Phe Phe Arg Ser Gly Lys
                245                 250                 255

Tyr Asp Leu Asp Phe Lys Ser Pro Asp Asp Pro Ser Arg Tyr Ile Ser
            260                 265                 270

Pro Asp Gln Leu Ala Asp Leu Tyr Lys Ser Phe Ile Lys Asp Tyr Pro
        275                 280                 285

Val Val Ser Ile Glu Asp Pro Phe Asp Gln Asp Asp Trp Gly Ala Trp
    290                 295                 300

Gln Lys Phe Thr Ala Ser Ala Gly Ile Gln Val Val Gly Asp Asp Leu
305                 310                 315                 320

Thr Val Thr Asn Pro Lys Arg Ile Ala Lys Ala Val Asn Glu Lys Ser
                325                 330                 335

Cys Asn Cys Leu Leu Leu Lys Val Asn Gln Ile Gly Ser Val Thr Glu
            340                 345                 350

Ser Leu Gln Ala Cys Lys Leu Ala Gln Ala Asn Gly Trp Gly Val Met
        355                 360                 365

Val Ser His Arg Ser Gly Glu Thr Glu Asp Thr Phe Ile Ala Asp Leu

    370                 375                 380

Val Val Gly Leu Cys Thr Gly Gln Ile Lys Thr Gly Ala Pro Cys Arg
385                 390                 395                 400

Ser Glu Arg Leu Ala Lys Tyr Asn Gln Leu Leu Arg Ile Glu Glu Glu
                405                 410                 415

Leu Gly Ser Lys Ala Lys Phe Ala Gly Arg Asn Phe Arg Asn Pro Leu
            420                 425                 430

Ala Lys

<210> 4
<211> 341
<212> PRT
<213> Homo sapiens

<300>
<308> Swissprot/P16220
<309> 2003-09-15
<313> (1)..(341)

<400> 4
Met Thr Met Glu Ser Gly Ala Glu Asn Gln Gln Ser Gly Asp Ala Ala
1               5                   10                  15

Val Thr Glu Ala Glu Asn Gln Gln Met Thr Val Gln Ala Gln Pro Gln
            20                  25                  30
Ile Ala Thr Leu Ala Gln Val Ser Met Pro Ala Ala His Ala Thr Ser
        35                  40                  45
Ser Ala Pro Thr Val Thr Leu Val Gln Leu Pro Asn Gly Gln Thr Val
    50                  55                  60
Gln Val His Gly Val Ile Gln Ala Ala Gln Pro Ser Val Ile Gln Ser
65                  70                  75                  80
Pro Gln Val Gln Thr Val Gln Ser Ser Cys Lys Asp Leu Lys Arg Leu
                85                  90                  95
Phe Ser Gly Thr Gln Ile Ser Thr Ile Ala Glu Ser Glu Asp Ser Gln
            100                 105                 110
Glu Ser Val Asp Ser Val Thr Asp Ser Gln Lys Arg Arg Glu Ile Leu
        115                 120                 125
Ser Arg Arg Pro Ser Tyr Arg Lys Ile Leu Asn Asp Leu Ser Ser Asp
    130                 135                 140
Ala Pro Gly Val Pro Arg Ile Glu Glu Glu Lys Ser Glu Glu Glu Thr
145                 150                 155                 160
Ser Ala Pro Ala Ile Thr Thr Val Thr Val Pro Thr Pro Ile Tyr Gln
                165                 170                 175

Thr Ser Ser Gly Gln Tyr Ile Ala Ile Thr Gln Gly Gly Ala Ile Gln
            180                 185                 190

Leu Ala Asn Asn Gly Thr Asp Gly Val Gln Gly Leu Gln Thr Leu Thr
        195                 200                 205

Met Thr Asn Ala Ala Ala Thr Gln Pro Gly Thr Thr Ile Leu Gln Tyr
    210                 215                 220

Ala Gln Thr Thr Asp Gly Gln Gln Ile Leu Val Pro Ser Asn Gln Val
225                 230                 235                 240

Val Val Gln Ala Ala Ser Gly Asp Val Gln Thr Tyr Gln Ile Arg Thr
                245                 250                 255

Ala Pro Thr Ser Thr Ile Ala Pro Gly Val Val Met Ala Ser Ser Pro
            260                 265                 270

Ala Leu Pro Thr Gln Pro Ala Glu Glu Ala Ala Arg Lys Arg Glu Val
        275                 280                 285

Arg Leu Met Lys Asn Arg Glu Ala Ala Arg Glu Cys Arg Arg Lys Lys
    290                 295                 300

Lys Glu Tyr Val Lys Cys Leu Glu Asn Arg Val Ala Val Leu Glu Asn
305                 310                 315                 320

Gln Asn Lys Thr Leu Ile Glu Glu Leu Lys Ala Leu Lys Asp Leu Tyr

                325                 330                 335

Cys His Lys Ser Asp
            340

<210> 5
<211> 254
<212> PRT
<213> Homo sapiens

<300>
<308> Swissprot/P18669
<309> 2003-09-15
<313> (1)..(254)

<400> 5
Met Ala Ala Tyr Lys Leu Val Leu Ile Arg His Gly Glu Ser Ala Trp
1               5                   10                  15

Asn Leu Glu Asn Arg Phe Ser Gly Trp Tyr Asp Ala Asp Leu Ser Pro
            20                  25                  30

Ala Gly His Glu Glu Ala Lys Arg Gly Gly Gln Ala Leu Arg Asp Ala
        35                  40                  45

Gly Tyr Glu Phe Asp Ile Cys Phe Thr Ser Val Gln Lys Arg Ala Ile
    50                  55                  60

Arg Thr Leu Trp Thr Val Leu Asp Ala Ile Asp Gln Met Trp Leu Pro
65                  70                  75                  80

Val Val Arg Thr Trp Arg Leu Asn Glu Arg His Tyr Gly Gly Leu Thr
                85                  90                  95

Gly Leu Asn Lys Ala Glu Thr Ala Ala Lys His Gly Glu Ala Gln Val
            100                 105                 110

Lys Ile Trp Arg Arg Ser Tyr Asp Val Pro Pro Pro Pro Met Glu Pro
        115                 120                 125

Asp His Pro Phe Tyr Ser Asn Ile Ser Lys Asp Arg Arg Tyr Ala Asp
    130                 135                 140

Leu Thr Glu Asp Gln Leu Pro Ser Cys Glu Ser Leu Lys Asp Thr Ile
145                 150                 155                 160

Ala Arg Ala Leu Pro Phe Trp Asn Glu Glu Ile Val Pro Gln Ile Lys
                165                 170                 175

Glu Gly Lys Arg Val Leu Ile Ala Ala His Gly Asn Ser Leu Arg Gly
            180                 185                 190

Ile Val Lys His Leu Glu Gly Leu Ser Glu Glu Ala Ile Met Glu Leu
        195                 200                 205

Asn Leu Pro Thr Gly Ile Pro Ile Val Tyr Glu Leu Asp Lys Asn Leu
    210                 215                 220

Lys Pro Ile Lys Pro Met Gln Phe Leu Gly Asp Glu Glu Thr Val Arg
225                 230                 235                 240

Lys Ala Met Glu Ala Val Ala Ala Gln Gly Lys Ala Lys Lys
                245                 250

<210>  6
<211>  220
<212>  PRT
<213>  Homo sapiens

<300>
<308>  Swissprot/P20336
<309>  2003-09-15
<313>  (1)..(220)

<400>  6
Met Ala Ser Ala Thr Asp Ser Arg Tyr Gly Gln Lys Glu Ser Ser Asp
1               5                   10                  15

Gln Asn Phe Asp Tyr Met Phe Lys Ile Leu Ile Ile Gly Asn Ser Ser
            20                  25                  30

Val Gly Lys Thr Ser Phe Leu Phe Arg Tyr Ala Asp Asp Ser Phe Thr
        35                  40                  45

Pro Ala Phe Val Ser Thr Val Gly Ile Asp Phe Lys Val Lys Thr Ile

    50                  55                  60

Tyr Arg Asn Asp Lys Arg Ile Lys Leu Gln Ile Trp Asp Thr Ala Gly
65                  70                  75                  80

Gln Glu Arg Tyr Arg Thr Ile Thr Thr Ala Tyr Tyr Arg Gly Ala Met
                85                  90                  95

Gly Phe Ile Leu Met Tyr Asp Ile Thr Asn Glu Glu Ser Phe Asn Ala
            100                 105                 110

Val Gln Asp Trp Ser Thr Gln Ile Lys Thr Tyr Ser Trp Asp Asn Ala
        115                 120                 125

Gln Val Leu Leu Val Gly Asn Lys Cys Asp Met Glu Asp Glu Arg Val
    130                 135                 140

Val Ser Ser Glu Arg Gly Arg Gln Leu Ala Asp His Leu Gly Phe Glu
145                 150                 155                 160

Phe Phe Glu Ala Ser Ala Lys Asp Asn Ile Asn Val Lys Gln Thr Phe
                165                 170                 175

Glu Arg Leu Val Asp Val Ile Cys Glu Lys Met Ser Glu Ser Leu Asp
            180                 185                 190

Thr Ala Asp Pro Ala Val Thr Gly Ala Lys Gln Gly Pro Gln Leu Ser
        195                 200                 205

Asp Gln Gln Val Pro Pro His Gln Asp Cys Ala Cys
    210                 215                 220

<210> 7
<211> 206
<212> PRT
<213> Homo sapiens

<300>
<308> Swissprot/P11233
<309> 2002-06-15
<313> (1)..(206)

<400> 7
<210> 8
<211> 557
<212> PRT

<213>  Homo sapiens

<300>

<308>  Swissprot/P49023

<309>  2003-09-15

<313>  (1)..(557)

<400>  8

Met Asp Asp Leu Asp Ala Leu Leu Ala Asp Leu Glu Ser Thr Thr Ser
1               5                   10                  15

His Ile Ser Lys Arg Pro Val Phe Leu Ser Glu Glu Thr Pro Tyr Ser
            20                  25                  30

Tyr Pro Thr Gly Asn His Thr Tyr Gln Glu Ile Ala Val Pro Pro Pro
        35                  40                  45

Val Pro Pro Pro Pro Ser Ser Glu Ala Leu Asn Gly Thr Ile Leu Asp
    50                  55                  60

Pro Leu Asp Gln Trp Gln Pro Ser Gly Ser Arg Phe Ile His Gln Gln
65                  70                  75                  80

Pro Gln Ser Ser Ser Pro Val Tyr Gly Ser Ser Ala Lys Thr Ser Ser
                85                  90                  95

Val Ser Asn Pro Gln Asp Ser Val Gly Ser Pro Cys Ser Arg Val Gly
            100                 105                 110

Glu Glu Glu His Val Tyr Ser Phe Pro Asn Lys Gln Lys Ser Ala Glu
        115                 120                 125
Pro Ser Pro Thr Val Met Ser Thr Ser Leu Gly Ser Asn Leu Ser Glu
    130                 135                 140
Leu Asp Arg Leu Leu Leu Glu Leu Asn Ala Val Gln His Asn Pro Pro
145                 150                 155                 160
Gly Phe Pro Ala Asp Glu Ala Asn Ser Ser Pro Pro Leu Pro Gly Ala
                165                 170                 175
Leu Ser Pro Leu Tyr Gly Val Pro Glu Thr Asn Ser Pro Leu Gly Gly
            180                 185                 190
Lys Ala Gly Pro Leu Thr Lys Glu Lys Pro Lys Arg Asn Gly Gly Arg
        195                 200                 205
Gly Leu Glu Asp Val Arg Pro Ser Val Glu Ser Leu Leu Asp Glu Leu
    210                 215                 220
Glu Ser Ser Val Pro Ser Pro Val Pro Ala Ile Thr Val Asn Gln Gly
225                 230                 235                 240
Glu Met Ser Ser Pro Gln Arg Val Thr Ser Thr Gln Gln Gln Thr Arg
                245                 250                 255
Ile Ser Ala Ser Ser Ala Thr Arg Glu Leu Asp Glu Leu Met Ala Ser
            260                 265                 270

Leu Ser Asp Phe Lys Phe Met Ala Gln Gly Lys Thr Gly Ser Ser Ser
        275                 280                 285
Pro Pro Gly Gly Pro Pro Lys Pro Gly Ser Gln Leu Asp Ser Met Leu
    290                 295                 300
Gly Ser Leu Gln Ser Asp Leu Asn Lys Leu Gly Val Ala Thr Val Ala
305                 310                 315                 320
Lys Gly Val Cys Gly Ala Cys Lys Lys Pro Ile Ala Gly Gln Val Val
                325                 330                 335
Thr Ala Met Gly Lys Thr Trp His Pro Glu His Phe Val Cys Thr His
            340                 345                 350
Cys Gln Glu Glu Ile Gly Ser Arg Asn Phe Phe Glu Arg Asp Gly Gln
        355                 360                 365
Pro Tyr Cys Glu Lys Asp Tyr His Asn Leu Phe Ser Pro Arg Cys Tyr
    370                 375                 380
Tyr Cys Asn Gly Pro Ile Leu Asp Lys Val Val Thr Ala Leu Asp Arg
385                 390                 395                 400
Thr Trp His Pro Glu His Phe Phe Cys Ala Gln Cys Gly Ala Phe Phe
                405                 410                 415
Gly Pro Glu Gly Phe His Glu Lys Asp Gly Lys Ala Tyr Cys Arg Lys

            420                 425                 430

Asp Tyr Phe Asp Met Phe Ala Pro Lys Cys Gly Gly Cys Ala Arg Ala

        435                 440                 445

Ile Leu Glu Asn Tyr Ile Ser Ala Leu Asn Thr Leu Trp His Pro Glu

    450                 455                 460

Cys Phe Val Cys Arg Glu Cys Phe Thr Pro Phe Val Asn Gly Ser Phe

465                 470                 475                 480

Phe Glu His Asp Gly Gln Pro Tyr Cys Glu Val His Tyr His Glu Arg

                485                 490                 495

Arg Gly Ser Leu Cys Ser Gly Cys Gln Lys Pro Ile Thr Gly Arg Cys

            500                 505                 510

Ile Thr Ala Met Ala Lys Lys Phe His Pro Glu His Phe Val Cys Ala

        515                 520                 525

Phe Cys Leu Lys Gln Leu Asn Lys Gly Thr Phe Lys Glu Gln Asn Asp

    530                 535                 540

Lys Pro Tyr Cys Gln Asn Cys Phe Leu Lys Leu Phe Cys

545                 550                 555

<210> 9

<211> 556

<212>  PRT

<213>  Homo sapiens

<300>

<308>  Swissprot/P17987

<309>  2003-09-15

<313>  (1)..(556)

<400>  9

Met Glu Gly Pro Leu Ser Val Phe Gly Asp Arg Ser Thr Gly Glu Thr
1               5                   10                  15

Ile Arg Ser Gln Asn Val Met Ala Ala Ala Ser Ile Ala Asn Ile Val
            20                  25                  30

Lys Ser Ser Leu Gly Pro Val Gly Leu Asp Lys Met Leu Val Asp Asp
        35                  40                  45

Ile Gly Asp Val Thr Ile Thr Asn Asp Gly Ala Thr Ile Leu Lys Leu
    50                  55                  60

Leu Glu Val Glu His Pro Ala Ala Lys Val Leu Cys Glu Leu Ala Asp
65                  70                  75                  80

Leu Gln Asp Lys Glu Val Gly Asp Gly Thr Thr Ser Val Val Ile Ile
                85                  90                  95

Ala Ala Glu Leu Leu Lys Asn Ala Asp Glu Leu Val Lys Gln Lys Ile
            100                 105                 110

His Pro Thr Ser Val Ile Ser Gly Tyr Arg Leu Ala Cys Lys Glu Ala
        115                 120                 125
Val Arg Tyr Ile Asn Glu Asn Leu Ile Val Asn Thr Asp Glu Leu Gly
    130                 135                 140
Arg Asp Cys Leu Ile Asn Ala Ala Lys Thr Ser Met Ser Ser Lys Ile
145                 150                 155                 160
Ile Gly Ile Asn Gly Asp Phe Phe Ala Asn Met Val Val Asp Ala Val
                165                 170                 175
Leu Ala Ile Lys Tyr Thr Asp Ile Arg Gly Gln Pro Arg Tyr Pro Val
            180                 185                 190
Asn Ser Val Asn Ile Leu Lys Ala His Gly Arg Ser Gln Met Glu Ser
        195                 200                 205
Met Leu Ile Ser Gly Tyr Ala Leu Asn Cys Val Val Gly Ser Gln Gly
    210                 215                 220
Met Pro Lys Arg Ile Val Asn Ala Lys Ile Ala Cys Leu Asp Phe Ser
225                 230                 235                 240
Leu Gln Lys Thr Lys Met Lys Leu Gly Val Gln Val Val Ile Thr Asp
                245                 250                 255
Pro Glu Lys Leu Asp Gln Ile Arg Gln Arg Glu Ser Asp Ile Thr Lys

            260                 265                 270

Glu Arg Ile Gln Lys Ile Leu Ala Thr Gly Ala Asn Val Ile Leu Thr
        275                 280                 285

Thr Gly Gly Ile Asp Asp Met Cys Leu Lys Tyr Phe Val Glu Ala Gly
    290                 295                 300

Ala Met Ala Val Arg Arg Val Leu Lys Arg Asp Leu Lys Arg Ile Ala
305                 310                 315                 320

Lys Ala Ser Gly Ala Thr Ile Leu Ser Thr Leu Ala Asn Leu Glu Gly
                325                 330                 335

Glu Glu Thr Phe Glu Ala Ala Met Leu Gly Gln Ala Glu Glu Val Val
            340                 345                 350

Gln Glu Arg Ile Cys Asp Asp Glu Leu Ile Leu Ile Lys Asn Thr Lys
        355                 360                 365

Ala Arg Thr Ser Ala Ser Ile Ile Leu Arg Gly Ala Asn Asp Phe Met
    370                 375                 380

Cys Asp Glu Met Glu Arg Ser Leu His Asp Ala Leu Cys Val Val Lys
385                 390                 395                 400

Arg Val Leu Glu Ser Lys Ser Val Val Pro Gly Gly Gly Ala Val Glu
                405                 410                 415

Ala Ala Leu Ser Ile Tyr Leu Glu Asn Tyr Ala Thr Ser Met Gly Ser
            420                 425                 430

Arg Glu Gln Leu Ala Ile Ala Glu Phe Ala Arg Ser Leu Leu Val Ile
        435                 440                 445

Pro Asn Thr Leu Ala Val Asn Ala Ala Gln Asp Ser Thr Asp Leu Val
    450                 455                 460

Ala Lys Leu Arg Ala Phe His Asn Glu Ala Gln Val Asn Pro Glu Arg
465                 470                 475                 480

Lys Asn Leu Lys Trp Ile Gly Leu Asp Leu Ser Asn Gly Lys Pro Arg
                485                 490                 495

Asp Asn Lys Gln Ala Gly Val Phe Glu Pro Thr Ile Val Lys Val Lys
            500                 505                 510

Ser Leu Lys Phe Ala Thr Glu Ala Ala Ile Thr Ile Leu Arg Ile Asp
        515                 520                 525

Asp Leu Ile Lys Leu His Pro Glu Ile Leu Arg Ile Lys His Gly Ser
    530                 535                 540

Tyr Glu Asp Ala Val His Ser Gly Ala Leu Asn Asp
545                 550                 555

<210> 10

<211>  505

<212>  PRT

<213>  Homo sapiens

<300>

<308>  Swissprot/P30101

<309>  2003-09-15

<313>  (1)..(505)

<400>  10

Met Arg Leu Arg Arg Leu Ala Leu Phe Pro Gly Val Ala Leu Leu Leu
1               5                   10                  15

Ala Ala Ala Arg Leu Ala Ala Ala Ser Asp Val Leu Glu Leu Thr Asp
            20                  25                  30

Asp Asn Phe Glu Ser Arg Ile Ser Asp Thr Gly Ser Ala Gly Leu Met
        35                  40                  45

Leu Val Glu Phe Phe Ala Pro Trp Cys Gly His Cys Lys Arg Leu Ala
    50                  55                  60

Pro Glu Tyr Glu Ala Ala Ala Thr Arg Leu Lys Gly Ile Val Pro Leu
65                  70                  75                  80

Ala Lys Val Asp Cys Thr Ala Asn Thr Asn Thr Cys Asn Lys Tyr Gly
                85                  90                  95

Val Ser Gly Tyr Pro Thr Leu Lys Ile Phe Arg Asp Gly Glu Glu Ala

            100                 105                 110

Gly Ala Tyr Asp Gly Pro Arg Thr Ala Asp Gly Ile Val Ser His Leu
        115                 120                 125

Lys Lys Gln Ala Gly Pro Ala Ser Val Pro Leu Arg Thr Glu Glu Glu
    130                 135                 140

Phe Lys Lys Phe Ile Ser Asp Lys Asp Ala Ser Ile Val Gly Phe Phe
145                 150                 155                 160

Asp Asp Ser Phe Ser Glu Ala His Ser Glu Phe Leu Lys Ala Ala Ser
                165                 170                 175

Asn Leu Arg Asp Asn Tyr Arg Phe Ala His Thr Asn Val Glu Ser Leu
            180                 185                 190

Val Asn Glu Tyr Asp Asp Asn Gly Glu Gly Ile Ile Leu Phe Arg Pro
        195                 200                 205

Ser His Leu Thr Asn Lys Phe Glu Asp Lys Thr Val Ala Tyr Thr Glu
    210                 215                 220

Gln Lys Met Thr Ser Gly Lys Ile Lys Lys Phe Ile Gln Glu Asn Ile
225                 230                 235                 240

Phe Gly Ile Cys Pro His Met Thr Glu Asp Asn Lys Asp Leu Ile Gln
                245                 250                 255

Gly Lys Asp Leu Leu Ile Ala Tyr Tyr Asp Val Asp Tyr Glu Lys Asn
            260                 265                 270
Ala Lys Gly Ser Asn Tyr Trp Arg Asn Arg Val Met Met Val Ala Lys
        275                 280                 285
Lys Phe Leu Asp Ala Gly His Lys Leu Asn Phe Ala Val Ala Ser Arg
    290                 295                 300
Lys Thr Phe Ser His Glu Leu Ser Asp Phe Gly Leu Glu Ser Thr Ala
305                 310                 315                 320
Gly Glu Ile Pro Val Val Ala Ile Arg Thr Ala Lys Gly Glu Lys Phe
                325                 330                 335
Val Met Gln Glu Glu Phe Ser Arg Asp Gly Lys Ala Leu Glu Arg Phe
            340                 345                 350
Leu Gln Asp Tyr Phe Asp Gly Asn Leu Lys Arg Tyr Leu Lys Ser Glu
        355                 360                 365
Pro Ile Pro Glu Ser Asn Asp Gly Pro Val Lys Val Val Val Ala Glu
    370                 375                 380
Asn Phe Asp Glu Ile Val Asn Asn Glu Asn Lys Asp Val Leu Ile Glu
385                 390                 395                 400
Phe Tyr Ala Pro Trp Cys Gly His Cys Lys Asn Leu Glu Pro Lys Tyr
                405                 410                 415

Lys Glu Leu Gly Glu Lys Leu Ser Lys Asp Pro Asn Ile Val Ile Ala
            420                 425                 430

Lys Met Asp Ala Thr Ala Asn Asp Val Pro Ser Pro Tyr Glu Val Arg
        435                 440                 445

Gly Phe Pro Thr Ile Tyr Phe Ser Pro Ala Asn Lys Lys Leu Asn Pro
    450                 455                 460

Lys Lys Tyr Glu Gly Gly Arg Glu Leu Ser Asp Phe Ile Ser Tyr Leu
465                 470                 475                 480

Gln Arg Glu Ala Thr Asn Pro Pro Val Ile Gln Glu Glu Lys Pro Lys
                485                 490                 495

Lys Lys Lys Lys Ala Gln Glu Asp Leu
            500                 505

<210> 11
<211> 406
<212> PRT
<213> Homo sapiens

<300>
<308> Swissprot/P04765
<309> 2003-09-15
<313> (1)..(406)

<400> 11

Met Ser Ala Ser Gln Asp Ser Arg Ser Arg Asp Asn Gly Pro Asp Gly

1               5                   10                  15

Met Glu Pro Glu Gly Val Ile Glu Ser Asn Trp Asn Glu Ile Val Asp

            20                  25                  30

Ser Phe Asp Asp Met Asn Leu Ser Glu Ser Leu Leu Arg Gly Ile Tyr

        35                  40                  45

Ala Tyr Gly Phe Glu Lys Pro Ser Ala Ile Gln Gln Arg Ala Ile Leu

    50                  55                  60

Pro Cys Ile Lys Gly Tyr Asp Val Ile Ala Gln Ala Gln Ser Gly Thr

65                  70                  75                  80

Gly Lys Thr Ala Thr Phe Ala Ile Ser Ile Leu Gln Gln Ile Glu Leu

                85                  90                  95

Asp Leu Lys Ala Thr Gln Ala Leu Val Leu Ala Pro Thr Arg Glu Leu

            100                 105                 110

Ala Gln Gln Ile Gln Lys Val Val Met Ala Leu Gly Asp Tyr Met Gly

        115                 120                 125

Ala Ser Cys His Ala Cys Ile Gly Gly Thr Asn Val Arg Ala Glu Val

    130                 135                 140

Gln Lys Leu Gln Met Glu Ala Pro His Ile Ile Val Gly Thr Pro Gly
145                 150                 155                 160

Arg Val Phe Asp Met Leu Asn Arg Arg Tyr Leu Ser Pro Lys Tyr Ile
                165                 170                 175

Lys Met Phe Val Leu Asp Glu Ala Asp Glu Met Leu Ser Arg Gly Phe
            180                 185                 190

Lys Asp Gln Ile Tyr Asp Ile Phe Gln Lys Leu Asn Ser Asn Thr Gln
        195                 200                 205

Val Val Leu Leu Ser Ala Thr Met Pro Ser Asp Val Leu Glu Val Thr
    210                 215                 220

Lys Lys Phe Met Arg Asp Pro Ile Arg Ile Leu Val Lys Lys Glu Glu
225                 230                 235                 240

Leu Thr Leu Glu Gly Ile Arg Gln Phe Tyr Ile Asn Val Glu Arg Glu
                245                 250                 255

Glu Trp Lys Leu Asp Thr Leu Cys Asp Leu Tyr Glu Thr Leu Thr Ile
            260                 265                 270

Thr Gln Ala Val Ile Phe Ile Asn Thr Arg Arg Lys Val Asp Trp Leu
        275                 280                 285

Thr Glu Lys Met His Ala Arg Asp Phe Thr Val Ser Ala Met His Gly
    290                 295                 300

Asp Met Asp Gln Lys Glu Arg Asp Val Ile Met Arg Glu Phe Arg Ser
305                 310                 315                 320

Gly Ser Ser Arg Val Leu Ile Thr Thr Asp Leu Leu Ala Arg Gly Ile
                325                 330                 335

Asp Val Gln Gln Val Ser Leu Val Ile Asn Tyr Asp Leu Pro Thr Asn
            340                 345                 350

Arg Glu Asn Tyr Ile His Arg Ile Gly Arg Gly Gly Arg Phe Gly Arg
        355                 360                 365

Lys Gly Val Ala Ile Asn Met Val Thr Glu Glu Asp Lys Arg Thr Leu
    370                 375                 380

Arg Asp Ile Glu Thr Phe Tyr Asn Thr Ser Ile Glu Glu Met Pro Leu
385                 390                 395                 400

Asn Val Ala Asp Leu Ile
                405

<210>  12
<211>  317
<212>  PRT
<213>  Homo sapiens

<300>

<308>  Swissprot/P25388

<309>  2003-09-15

<313>  (1)..(317)

<400>  12

<210> 13
<211> 321
<212> PRT
<213> Homo sapiens

<300>
<308> Swissprot/P22087
<309> 2003-09-15
<313> (1)..(321)

<400> 13
Met Lys Pro Gly Phe Ser Pro Arg Gly Gly Gly Phe Gly Gly Arg Gly
1               5                   10                  15

Gly Phe Gly Asp Arg Gly Gly Arg Gly Gly Arg Gly Gly Phe Gly Gly
20. 25 30

Gly Arg Gly Arg Gly Gly Gly Phe Arg Gly Arg Gly Arg Gly Gly Gly
35 40 45

Gly Gly Gly Gly Gly Gly Gly Gly Gly Gly Arg Gly Gly Gly Gly Phe

50                  55                  60

His Ser Gly Gly Asn Arg Gly Arg Gly Arg Gly Gly Lys Arg Gly Asn
65                  70                  75                  80

Gln Ser Gly Lys Asn Val Met Val Glu Pro His Arg His Glu Gly Val
                85                  90                  95

Phe Ile Cys Arg Gly Lys Glu Asp Ala Leu Val Thr Lys Asn Leu Val
            100                 105                 110

Pro Gly Glu Ser Val Tyr Gly Glu Lys Arg Val Ser Ile Ser Glu Gly
        115                 120                 125

Asp Asp Lys Ile Glu Tyr Arg Ala Trp Asn Pro Phe Arg Ser Lys Leu
    130                 135                 140

Ala Ala Ala Ile Leu Gly Gly Val Asp Gln Ile His Ile Lys Pro Gly
145                 150                 155                 160

Ala Lys Val Leu Tyr Leu Gly Ala Ala Ser Gly Thr Thr Val Ser His
                165                 170                 175

Val Ser Asp Ile Val Gly Pro Asp Gly Leu Val Tyr Ala Val Glu Phe
            180                 185                 190

Ser His Arg Ser Gly Arg Asp Leu Ile Asn Leu Ala Lys Lys Arg Thr
        195                 200                 205

Asn Ile Ile Pro Val Ile Glu Asp Ala Arg His Pro His Lys Tyr Arg
210 215 220

Met Leu Ile Ala Met Val Asp Val Ile Phe Ala Asp Val Ala Gln Pro
225 230 235 240

Asp Gln Thr Arg Ile Val Ala Leu Asn Ala His Thr Phe Leu Arg Asn
245 250 255

Gly Gly His Phe Val Ile Ser Ile Lys Ala Asn Cys Ile Asp Ser Thr
260 265 270

Ala Ser Ala Glu Ala Val Phe Ala Ser Glu Val Lys Lys Met Gln Gln
275 280 285

Glu Asn Met Lys Pro Gln Glu Gln Leu Thr Leu Glu Pro Tyr Glu Arg
290 295 300

Asp His Ala Val Val Val Gly Val Tyr Arg Pro Pro Pro Lys Val Lys
305 310 315 320

Asn

<210> 14
<211> 267
<212> PRT
<213> Homo sapiens

<300>

<308>  Swissprot/P02647

<309>  2003-09-15

<313>  (1)..(267)

<400>  14

<210> 15

<211> 272

<212> PRT

<213> Homo sapiens

<300>

<308> Swissprot/P35232

<309> 2003-09-15

<313> (1)..(272)

<400> 15
   Met Ala Ala Lys Val Phe Glu Ser Ile Gly Lys Phe Gly Leu Ala Leu
   1               5                   10                  15
   Ala Val Ala Gly Gly Val Val Asn Ser Ala Leu Tyr Asn Val Asp Ala
               20                  25                  30
   Gly His Arg Ala Val Ile Phe Asp Arg Phe Arg Gly Val Gln Asp Ile
           35                  40                  45
   Val Val Gly Glu Gly Thr His Phe Leu Ile Pro Trp Val Gln Lys Pro
       50                  55                  60
   Ile Ile Phe Asp Cys Arg Ser Arg Pro Arg Asn Val Pro Val Ile Thr
   65                  70                  75                  80
   Gly Ser Lys Asp Leu Gln Asn Val Asn Ile Thr Leu Arg Ile Leu Phe
                   85                  90                  95
   Arg Pro Val Ala Ser Gln Leu Pro Arg Ile Phe Thr Ser Ile Gly Glu
   100 105 110
   Asp Tyr Asp Glu Arg Val Leu Pro Ser Ile Thr Thr Glu Ile Leu Lys
   115 120 125
   Ser Val Val Ala Arg Phe Asp Ala Gly Glu Leu Ile Thr Gln Arg Glu
   130 135 140
   Leu Val Ser Arg Gln Val Ser Asp Asp Leu Thr Glu Arg Ala Ala Thr
   145 150 155 160
   Phe Gly Leu Ile Leu Asp Asp Val Ser Leu Thr His Leu Thr Phe Gly
   165 170 175
   Lys Glu Phe Thr Glu Ala Val Glu Ala Lys Gln Val Ala Gln Gln Glu
   180 185 190
   Ala Glu Arg Ala Arg Phe Val Val Glu Lys Ala Glu Gln Gln Lys Lys
   195 200 205
   Ala Ala Ile Ile Ser Ala Glu Gly Asp Ser Lys Ala Ala Glu Leu Ile
   210 215 220
   Ala Asn Ser Leu Ala Thr Ala Gly Asp Gly Leu Ile Glu Leu Arg Lys
   225 230 235 240
   Leu Glu Ala Ala Glu Asp Ile Ala Tyr Gln Leu Ser Arg Ser Arg Asn
   245 250 255
   Ile Thr Tyr Leu Pro Ala Gly Gln Ser Val Leu Leu Gln Leu Pro Gln
   260 265 270

<210> 16
<211> 193
<212> PRT
<213> Homo sapiens

<300>
<308> Swissprot/P06749
<309> 2003-09-15
<313> (1)..(193)

<400> 16
Met Ala Ala Ile Arg Lys Lys Leu Val Ile Val Gly Asp Gly Ala Cys
1               5                   10                  15

Gly Lys Thr Cys Leu Leu Ile Val Phe Ser Lys Asp Gln Phe Pro Glu
            20                  25                  30

Val Tyr Val Pro Thr Val Phe Glu Asn Tyr Val Ala Asp Ile Glu Val
        35                  40                  45

Asp Gly Lys Gln Val Glu Leu Ala Leu Trp Asp Thr Ala Gly Gln Glu
    50                  55                  60

Asp Tyr Asp Arg Leu Arg Pro Leu Ser Tyr Pro Asp Thr Asp Val Ile
65                  70                  75                  80

Leu Met Cys Phe Ser Ile Asp Ser Pro Asp Ser Leu Glu Asn Ile Pro
                85                  90                  95

Glu Lys Trp Thr Pro Glu Val Lys His Phe Cys Pro Asn Val Pro Ile
            100                 105                 110

Ile Leu Val Gly Asn Lys Lys Asp Leu Arg Asn Asp Glu His Thr Arg
        115                 120                 125

Arg Glu Leu Ala Lys Met Lys Gln Glu Pro Val Lys Pro Glu Glu Gly
    130                 135                 140

Arg Asp Met Ala Asn Arg Ile Gly Ala Phe Gly Tyr Met Glu Cys Ser
145                 150                 155                 160

Ala Lys Thr Lys Asp Gly Val Arg Glu Val Phe Glu Met Ala Thr Arg
                165                 170                 175

Ala Ala Leu Gln Ala Arg Arg Gly Lys Lys Lys Ser Gly Cys Leu Val
            180                 185                 190

Leu

<210>  17

<211>  1899

<212>  DNA

<213>  Homo sapiens


<300>

<308>  GenBank/NM_012341

<309>  2003-04-06

<313>  (1)..(1899)


<400>  17

atggcacatt acaacttcaa gaaaattacg gtggtgccgt ccgccaagga cttcatagac      60

ctcacgttgt cgaagactca acgaaagact ccaaccgtta ttcataaaca ttaccaaata     120

catcgcatta gacattttta catgagaaaa gtcaaattta ctcaacagaa ttaccatgat     180

agactttcac aaattctaac agatttcccc aaattggatg atattcatcc gttctatgct     240

gatttgatga atattctcta cgacaaggat cattacaagt tggctctggg gcaaataaat     300

attgccaaaa atttagtgga caatgttgct aaagattatg tgcgactgat gaagtatggc     360

gactctctct accgctgcaa acagctgaag cgtgcggccc tgggacggat gtgcacagtg     420

atcaagaggc agaagcagag tttggagtat ttggagcaag tgcgtcagca tttatcccgt     480

ttgccaacca ttgatccgaa taccaggacc ctgcttttgt gtgggtaccc aaatgttggg     540

aagtccagct tcatcaacaa ggtgacgaga gcagacgtgg atgtccagcc ctatgcgttc     600

acaaccaagt ctctgtttgt tgggcacatg gattataagt atctacgttg gcaggttgta     660

gacactcctg ggatcctgga ccaccctctg gaggatagga acaccatcga gatgcaggcc     720

atcactgccc tggcccacct ccgtgctgcg gtcctgtatg tgatggattt gtctgagcag     780

tgtgggcatg ggctgaggga gcagctagaa ctcttccaga acatcagacc tctcttcatc     840

aacaagcctc tcatagttgt agccaacaaa tgtgatgtga agagaatagc tgaactttct     900

gaagatgatc agaaaatatt tacagatttg cagtctgaag gattccctgt aatagagacc     960

agcaccctga ctgaggaagg tgttattaaa gttaaaacag aggcttgcga taggcttttg    1020

gctcatcgag tggaaaccaa aatgaaggga aataaagtga atgaggtgct gaatagactg    1080

cacctggcta tcccaaccag gagggacgat aaggagaggc cccctttcat ccctgaagga    1140

gtggtggctc gcaggaagag gatggaaact gaggagtcca ggaagaagag ggaacgagat 1200
cttgagctgg aaatgggaga tgattatatt ttggatcttc agaagtactg ggatttaatg 1260
aatttgtctg aaaaacatga taagatacca gaaatctggg aaggccataa tatagctgat 1320
tatattgatc cagccatcat gaagaaattg gaagaattag aaaaagaaga agagctgaga 1380
acagctgctg gagagtatga cagtgtatct gagagtgaag acgaagagat gctggaaatc 1440
cgacagctgg caaagcaaat tcgagagaaa aagaagttga aaattctgga gtccaaagaa 1500
aagaatacac agggacccag gatgccgcga actgctaaga aggttcagag gacagttttg 1560
gagaaggaga tgcgtagtct tggtgttgac atggacgata aagacgatgc ccattacgca 1620
gtccaggcaa gaagatcccg gagcatcact aggaaaagaa agcgggaaga ctctgctccc 1680
ccgtcctctg tggcccggag tgggagttgc tctcgaactc cacgtgacgt ttctggtctt 1740
agggatgtca agatggtgaa gaaagccaag actatgaaga atgctcagaa gaagatgaat 1800
cggttgggga agaaagggga ggcggataga cacgtgtttg atatgaagcc caagcacttg 1860
ctgtctggga agaggaaagc tggtaaaaag gacaggaga 1899

<210> 18
<211> 1674
<212> DNA
<213> Homo sapiens

<300>
<308> GenBank/X07674
<309> 1994-12-16
<313> (1)..(1674)

<400> 18
atgtaccgct acctgggcga agcgctgttg ctgtcccggg ccgggcccgc tgccctgggc 60
tcggcgtccg ccgactcggc cgcgttgctg ggctgggccc ggggacagcc cgccgccgcc 120
ccgcagccgg ggctggcatt ggccgcccgg cgccactaca gcgaggcggt ggccgaccgc 180

gaggacgacc ccaacttctt caagatggtg gagggcttct tcgatcgcgg cgccagcatc 240
gtggaggaca agctggtgga ggacctgagg acccgggaga gcgaggagca gaagcggaac 300
cgggtgcgcg gcatcctgcg gatcatcaag ccctgcaacc atgtgctgag tctctccttc 360
cccatccggc gcgacgacgg ctcctgggag gtcatcgaag gctaccgggc ccagcacagc 420
cagcaccgca cgccctgcaa gggaggtatc cgttacagca ctgatgtgag tgtagatgaa 480
gtaaaagctt tggcttctct gatgacatac aagtgtgcag tggttgatgt gccgtttggg 540
ggtgctaaag ctggtgttaa gatcaatccc aagaactata ctgataatga attggaaaag 600
atcacaagga ggttcaccat ggagctagca aaaaagggct ttattggtcc tggcattgat 660
gtgcctgctc cagacatgag cacaggtgag cgggagatgt cctggatcgc tgatacctat 720
gccagcacca tagggcacta tgatattaat gcacacgcct gtgttactgg taaacccatc 780
agccaagggg gaatccatgg acgcatctct gctactggcc gtggtgtctt ccatgggatt 840
gaaaatttca tcaatgaagc ttcttacatg agcattttag gaatgacacc agggtttgga 900
gataaaacat ttgttgttca gggatttggt aatgtgggcc tacactctat gagatattta 960
catcgttttg gtgctaaatg tattgctgtt ggtgagtctg atgggagtat atggaatcca 1020
gatggtattg acccaaagga actggaagac ttcaaattgc aacatgggtc cattctgggc 1080
ttccccaagg caaagcccta tgaaggaagc atcttggagg ccgactgtga catactgatc 1140
ccagctgcca gtgagaagca gttgaccaaa tccaacgcac ccagagtcaa agccaagatc 1200
attgctgaag gtgccaatgg gccaacaact ccagaagctg acaagatctt cctggagaga 1260
aacattatgg ttattccaga tctctacttg aatgctggag gagtgacagt atcttacttt 1320
gagtggctga agaatctaaa tcatgtcagc tatggccgtt tgaccttcaa atatgaaagg 1380
gattctaact accacttgct catgtctgtt caagagagtt tagaaagaaa atttggaaag 1440
catggtggaa ctattcccat tgtacccacg gcagagttcc aagacaggat atcgggtgca 1500
tctgagaaag acatcgtgca ctctggcttg gcatacacaa tggagcgttc tgccaggcaa 1560
attatgcgca cagccatgaa gtataacctg ggattggacc tgagaacagc tgcctatgtt 1620
aatgccattg agaaagtctt caaagtgtac aatgaagctg gtgtgacctt caca 1674

<210> 19
<211> 1302

<212> DNA

<213> Homo sapiens

<300>

<308> GenBank/BT007163

<309> 2003-05-13

<313> (1)..(1302)

<400> 19

atgtctattc tcaagatcca tgccagggag atctttgact ctcgcgggaa tcccactgtt 60

gaggttgatc tcttcacctc aaaaggtctc ttcagagctg ctgtgcccag tggtgcttca 120

actggtatct atgaggccct agagctccgg gacaatgata agactcgcta tatggggaag 180

ggtgtctcaa aggctgttga gcacatcaat aaaactattg cgcctgccct ggttagcaag 240

aaactgaacg tcacagaaca agagaagatt gacaaactga tgatcgagat ggatggaaca 300

gaaaataaat ctaagtttgg tgcgaacgcc attctggggg tgtcccttgc cgtctgcaaa 360

gctggtgccg ttgagaaggg ggtccccctg taccgccaca tcgctgactt ggctggcaac 420

tctgaagtca tcctgccagt cccggcgttc aatgtcatca atggcggttc tcatgctggc 480

aacaagctgg ccatgcagga gttcatgatc ctcccagtcg gtgcagcaaa cttcagggaa 540

gccatgcgca ttggagcaga ggtttaccac aacctgaaga atgtcatcaa ggagaaatat 600

gggaaagatg ccaccaatgt gggggatgaa ggcgggtttg ctcccaacat cctggagaat 660

aaagaaggcc tggagctgct gaagactgct attgggaaag ctggctacac tgataaggtg 720

gtcatcggca tggacgtagc ggcctccgag ttcttcaggt ctgggaagta tgacctggac 780

ttcaagtctc ccgatgaccc cagcaggtac atctcgcctg accagctggc tgacctgtac 840

aagtccttca tcaaggacta cccagtggtg tctatcgaag atccctttga ccaggatgac 900

tggggagctt ggcagaagtt cacagccagt gcaggaatcc aggtagtggg ggatgatctc 960

acagtgacca acccaaagag gatcgccaag gccgtgaacg agaagtcctg caactgcctc 1020

ctgctcaaag tcaaccagat tggctccgtg accgagtctc ttcaggcgtg caagctggcc 1080

caggccaatg gttggggcgt catggtgtct catcgttcgg gggagactga agataccttc 1140

atcgctgacc tggttgtggg gctgtgcact gggcagatca agactggtgc cccttgccga 1200

tctgagcgct tggccaagta caaccagctc ctcagaattg aagaggagct gggcagcaag    1260

gctaagtttg ccggcaggaa cttcagaaac cccttggcca ag                        1302

<210>  20

<211>  1023

<212>  DNA

<213>  Homo sapiens

<300>

<308>  GenBank/X55545

<309>  2000-10-04

<313>  (1)..(1023)

<400>  20

atgaccatgg aatctggagc cgagaaccag cagagtggag atgcagctgt aacagaagct      60

gaaaaccaac aaatgacagt tcaagcccag ccacagattg ccacattagc ccaggtatct     120

atgccagcag ctcatgcaac atcatctgct cccaccgtaa ctctagtaca gctgcccaat     180

gggcagacag ttcaagtcca tggagtcatt caggcggccc agccatcagt tattcagtct     240

ccacaagtcc aaacagttca gtcttcctgt aaggacttaa aaagactttt ctccggaaca     300

cagatttcaa ctattgcaga aagtgaagat tcacaggagt cagtggatag tgtaactgat     360

tcccaaaagc gaagggaaat tctttcaagg aggccttcct acaggaaaat tttgaatgac     420

ttatcttctg atgcaccagg agtgccaagg attgaagaag agaagtctga agaggagact     480

tcagcacctg ccatcaccac tgtaacggtg ccaactccaa tttaccaaac tagcagtgga     540

cagtatattg ccattaccca gggaggagca atacagctgg ctaacaatgg taccgatggg     600

gtacagggcc tgcaaacatt aaccatgacc aatgcagcag ccactcagcc gggtactacc     660

attctacagt atgcacagac cactgatgga cagcagatct tagtgcccag caaccaagtt     720

gttgttcaag ctgcctctgg agacgtacaa acataccaga ttcgcacagc acccactagc     780

actattgccc ctggagttgt tatggcatcc tccccagcac ttcctacaca gcctgctgaa     840

gaagcagcac gaaagagaga ggtccgtcta atgaagaaca gggaagcagc tcgagagtgt 900

cgtagaaaga agaaagaata tgtgaaatgt ttagaaaaca gagtggcagt gcttgaaaat 960

caaaacaaga cattgattga ggagctaaaa gcacttaagg acctttactg ccacaaatca 1020

gat 1023

<210> 21

<211> 762

<212> DNA

<213> Homo sapiens

<300>

<308> GenBank/J04173

<309> 1995-01-07

<313> (1)..(762)

<400> 21

atggccgcct acaaactggt gctgatccgg cacggcgaga gcgcatggaa cctggagaac 60

cgcttcagcg gctggtacga cgccgacctg agcccggcgg gccacgagga ggcgaagcgc 120

ggcgggcagg cgctacgaga tgctggctat gagtttgaca tctgcttcac ctcagtgcag 180

aagagagcga tccggaccct ctggacagtg ctagatgcca ttgatcagat gtggctgcca 240

gtggtgagga cttggcgcct caatgagcgg cactatgggg gtctaaccgg tctcaataaa 300

gcagaaactg ctgcaaagca tggtgaggcc caggtgaaga tctggaggcg ctcctatgat 360

gtcccaccac ctccgatgga gcccgaccat cctttctaca gcaacatcag taaggatcgc 420

aggtatgcag acctcacaga agatcagcta ccctcctgtg agagtctgaa ggatactatt 480

gccagagctc tgcccttctg gaatgaagaa atagttcccc agatcaagga ggggaaacgt 540

gtactgattg cagcccatgg caacagcctc cggggcattg tcaagcatct ggagggtctc 600

tctgaagagg ctatcatgga gctgaacctg ccgactggta ttcccattgt ctatgaattg 660

gacaagaact tgaagcctat caagcccatg cagtttctgg gggatgaaga gacggtgcgc 720

aaagccatgg aagctgtggc tgcccagggc aaggccaaga ag 762

<210> 22

<211> 660

<212> DNA

<213> Homo sapiens

<300>

<308> GenBank/NM_002826

<309> 2003-04-06

<313> (1)..(660)

<400> 22

atggcatccg ccacagactc gcgctatggg cagaaggagt cctcggatca gaacttcgac 60

tacatgttca agattctcat catcggcaac agcagcgtgg gcaagacgtc cttcctcttc 120

cgctatgctg acgactcgtt cacgcctgcc ttcgtcagca ccgtgggcat cgacttcaag 180

gtcaagacca tctatcgcaa cgacaagagg atcaagctgc agatctggga cacagcaggg 240

caagagcggt accggaccat caccaccgca tactaccggg gcgctatggg cttcatcctc 300

atgtatgaca tcaccaacga ggaatccttc aatgcagtgc aggactggtc cacccagatc 360

aagacctact catgggacaa tgcccaggtg ctgctggtag gaaacaagtg tgacatggag 420

gatgagcggg tggtgtcatc agaacgtggc cggcagctag ctgaccacct tgggttcgag 480

ttctttgagg caagcgccaa ggacaacatt aacgtcaagc agacctttga gcgcctggtg 540

gatgtcatct gcgagaagat gtccgagtcg ttggacacgg cggaccctgc ggtcacaggc 600

gccaagcagg gcccacagct cagtgaccag caggtgccac cgcaccagga ctgcgcctgc 660

<210> 23

<211> 618

<212>  DNA

<213>  Homo sapiens

<300>

<308>  GenBank/X15014

<309>  1993-09-12

<313>  (1)..(618)

<400>  23

atggctgcaa ataagcccaa gggtcagaat tctttggctt tacacaaagt catcatggtg      60

ggcagtggtg gcgtgggcaa gtcagctctg actctacagt tcatgtacga tgagtttgtg     120

gaggactatg agcctaccaa agcagacagc tatcggaaga aggtagtgct agatggggag     180

gaagtccaga tcgatatctt agatacagct gggcaggagg actacgctgc aattagagac     240

aactacttcc gaagtgggga ggggttcctc tgtgttttct ctattacaga aatggaatcc     300

tttgcagcta cagctgactt cagggagcag attttaagag taaaagaaga tgagaatgtt     360

ccatttctac tggttggtaa caaatcagat ttagaagata aaagacaggt ttctgtagaa     420

gaggcaaaaa acagagctga gcagtggaat gttaactacg tggaaacatc tgctaaaaca     480

cgagctaatg ttgacaaggt attttttgat ttaatgagag aaattcgagc gagaaagatg     540

gaagacagca aagaaaagaa tggaaaaaag aagaggaaaa gtttagccaa gagaatcaga     600

gaaagatgct gcatttta                                                   618

<210>  24

<211>  1671

<212>  DNA

<213>  Homo sapiens

<300>

<308>  GenBank/U14588

<309>  1995-03-10

<313>  (1)..(1671)

<400>  24

atggacgacc tcgacgccct gctggcggac ttggagtcta ccacctccca catctccaaa      60

cggcctgtgt tcttgtcgga ggagaccccc tactcatacc caactggaaa ccacacatac     120

caggagattg ccgtgccacc ccccgtcccc ccacccccgt ccagcgaggc cctcaatggc     180

acaatccttg accccttaga ccagtggcag cccagcggct cccgattcat ccaccagcag     240

cctcagtcct catcacctgt gtacggctcc agtgccaaaa cttccagtgt ctccaaccct     300

caggacagtg ttggctctcc gtgctcccga gtgggtgagg aggagcacgt ctacagcttc     360

cccaacaagc agaaatcagc tgagccttca cccaccgtaa tgagcacgtc cctgggcagc     420

aacctttctg aactcgaccg cctgctgctg gaactgaacg ctgtacagca taacccgcca     480

ggcttccctg cagatgaggc caactcaagc cccccgcttc ctggggccct gagccccctc     540

tatggtgtcc cagagactaa cagccccttg ggaggcaaag ctgggcccct gacgaaagag     600

aagcctaagc ggaatggggg ccggggcctg gaggacgtgc ggcccagtgt ggagagtctc     660

ttggatgaac tggagagctc cgtgcccagc cccgtccctg ccatcactgt gaaccagggc     720

gagatgagca gcccgcagcg cgtcacctcc acccaacagc agacacgcat ctcggcctcc     780

tctgccacca gggagctgga cgagctgatg gcttcgctgt cggatttcaa gttcatggcc     840

caggggaaga cagggagcag ctcaccccct gggggccccc cgaagcccgg gagccagctg     900

gacagcatgc tggggagcct gcagtctgac ctgaacaagc tgggggtcgc cacagtcgcc     960

aaaggagtct gcggggcctg caagaagccc atcgccgggc aggttgtgac cgccatgggg    1020

aagacgtggc accccgagca cttcgtctgc acccactgcc aggaggagat cggatcccgg    1080

aacttcttcg agcgggatgg acagccctac tgtgaaaagg actaccacaa cctcttctcc    1140

ccgcgctgct actactgcaa cggccccatc ctggataaag tggtgacagc ccttgaccgg    1200

acgtggcacc ctgaacactt cttctgtgca cagtgtggag ccttctttgg tcccgaaggg    1260

ttccacgaga aggacggcaa ggcctactgt cgcaaggact acttcgacat gttcgcaccc    1320

aagtgtggcg gctgcgcccg ggccatcctg gagaactata tctcagccct caacacgctg    1380

tggcatcctg agtgctttgt gtgccgggaa tgcttcacgc cattcgtgaa cggcagcttc    1440

ttcgagcacg acgggcagcc ctactgtgag gtgcactacc acgagcggcg cggctcgctg    1500

tgttctggct gccagaagcc catcaccggc cgctgcatca ccgccatggc caagaagttc 1560
caccccgagc acttcgtctg tgccttctgc ctcaagcagc tcaacaaggg caccttcaag 1620
gagcagaacg acaagcctta ctgtcagaac tgcttcctca agctcttctg c 1671

<210> 25
<211> 1668
<212> DNA
<213> Homo sapiens

<300>
<308> GenBAnk/NM_030752
<309> 2003-04-06
<313> (1)..(1668)

<400> 25
atggaggggc ctttgtccgt gttcggtgac cgcagcactg gggaaacgat ccgctcccaa 60
aacgttatgg ctgcagcttc gattgccaat attgtaaaaa gttctcttgg tccagttggc 120
ttggataaaa tgttggtgga tgatattggt gatgtaacca ttactaacga tggtgcaacc 180
atcctgaagt tactggaggt agaacatcct gcagctaaag ttctttgtga gctggctgat 240
ctgcaagaca aagaagttgg agatggaact acttcagtgg ttattattgc agcagaactc 300
ctaaaaaatg cagatgaatt agtcaaacag aaaattcatc ccacatcagt tattagtggc 360
tatcgacttg cttgcaagga agcagtgcgt tatatcaatg aaaacctaat tgttaacaca 420
gatgaactgg gaagagattg cctgattaat gctgctaaga catccatgtc ttccaaaatc 480
attgggataa atggtgattt ctttgctaac atggtagtag atgctgtact tgctattaaa 540
tacacagaca taagaggcca gccacgctat ccagtcaact ctgttaatat tttgaaagcc 600
catgggagaa gtcaaatgga gagtatgctc atcagtggct atgcactcaa ctgtgtggtg 660
ggatcccagg gcatgcccaa gagaatcgta aatgcaaaaa ttgcttgcct tgacttcagc 720
ctgcaaaaaa caaaaatgaa gcttggtgta caggtggtca ttacagaccc tgaaaaactg 780

gaccaaatta gacagagaga atcagatatc accaaggaga gaattcagaa gatcctggca 840
actggtgcca atgttattct aaccactggt ggaattgatg atatgtgtct gaagtatttt 900
gtggaggctg gtgctatggc agttagaaga gttttaaaaa gggaccttaa acgcattgcc 960
aaagcttctg gagcaactat tctgtcaacc ctggccaatt tggaaggtga agaaactttt 1020
gaagctgcaa tgttgggaca ggcagaagaa gtggtacagg agagaatttg tgatgatgag 1080
ctgatcttaa tcaaaaatac taaggctcgt acgtctgcat cgattatctt acgtggggca 1140
aatgatttca tgtgtgatga gatggagcgc tctttacatg atgcactttg tgtagtgaag 1200
agagttttgg agtcaaaatc tgtggttccc ggtgggggtg ctgtagaagc agccctttcc 1260
atataccttg aaaactatgc aaccagcatg gggtctcggg aacagcttgc gattgcagag 1320
tttgcaagat cacttcttgt tattcccaat acactagcag ttaatgctgc ccaggactcc 1380
acagatctgg ttgcaaaatt aagagctttt cataatgagg cccaggttaa cccagaacgt 1440
aaaaatctaa aatggattgg tcttgatttg agcaatggta aacctcgaga caacaaacaa 1500
gcaggggtgt ttgaaccaac catagttaaa gttaagagtt tgaaatttgc aacagaagct 1560
gcaatcacca ttcttcgaat tgatgatctt attaaattac atccagaaat ccttcggatt 1620
aaacatggaa gttatgaaga tgctgttcac tctggagccc ttaatgat 1668

<210> 26
<211> 1515
<212> DNA
<213> Homo sapiens

<300>
<308> GenBank/NM_005313
<309> 2003-04-06
<313> (1)..(1515)

<400> 26
atgcgcctcc gccgcctagc gctgttcccg ggtgtggcgc tgcttcttgc cgcggcccgc 60

ctcgccgctg cctccgacgt gctagaactc acggacgaca acttcgagag tcgcatctcc 120

gacacgggct ctgcgggcct catgctcgtc gagttcttcg ccccctggtg tggacactgc 180

aagagacttg cacctgagta tgaagctgca gctaccagat taaaaggaat agtcccatta 240

gcaaaggttg attgcactgc caacactaac acctgtaata aatatggagt cagtggatat 300

ccaaccctga agatatttag agatggtgaa gaagcaggtg cttatgatgg acctaggact 360

gctgatggaa ttgtcagcca cttgaagaag caggcaggac cagcttcagt gcctctcagg 420

actgaggaag aatttaagaa attcattagt gataaagatg cctctatagt aggttttttc 480

gatgattcat tcagtgaggc tcactccgag ttcctaaaag cagccagcaa cttgagggat 540

aactaccgat ttgcacatac gaatgttgag tctctggtga acgagtatga tgataatgga 600

gagggtatca tcttatttcg tccttcacat ctcactaaca agtttgagga caagactgtg 660

gcatatacag agcaaaaaat gaccagtggc aaaattaaaa agtttatcca ggaaaacatt 720

tttggtatct gccctcacat gacagaagac aataaagatt tgatacaggg caaggactta 780

cttattgctt actatgatgt ggactatgaa aagaacgcta aaggttccaa ctactggaga 840

aacagggtaa tgatggtggc aaagaaattc ctggatgctg ggcacaaact caactttgct 900

gtagctagcc gcaaaacctt tagccatgaa ctttctgatt ttggcttgga gagcactgct 960

ggagagattc ctgttgttgc tatcagaact gctaaaggag agaagtttgt catgcaggag 1020

gagttctcgc gtgatgggaa ggctctggag aggttcctgc aggattactt tgatggcaat 1080

ctgaagagat acctgaagtc tgaacctatc ccagagagca atgatgggcc tgtgaaggta 1140

gtggtagcag agaattttga tgaaatagtg aataatgaaa ataaagatgt gctgattgaa 1200

ttttatgccc cttggtgtgg tcactgtaag aacctggagc ccaagtataa agaacttggc 1260

gagaagctca gcaaagaccc aaatatcgtc atagccaaga tggatgccac agccaatgat 1320

gtgccttctc catatgaagt cagaggtttt cctaccatat acttctctcc agccaacaag 1380

aagctaaatc caaagaaata tgaaggtggc cgtgaattaa gtgattttat tagctatcta 1440

caaagagaag ctacaaaccc ccctgtaatt caagaagaaa aacccaagaa gaagaagaag 1500

gcacaggagg atctc 1515

<210> 27

<211> 1218

<212>  DNA
<213>  Homo sapiens

<300>
<308>  GenBank/NM_001416
<309>  2003-04-07
<313>  (1)..(1218)

<400>  27
atgtctgcga gccaggattc ccgatccaga gacaatggcc ccgatgggat ggagcccgaa      60
ggcgtcatcg agagtaactg gaatgagatt gttgacagct ttgatgacat gaacctctcg     120
gagtcccttc tccgtggcat ctacgcctat ggttttgaga agccctctgc catccagcag     180
cgagccattc taccttgtat caagggttat gatgtgattg ctcaagccca atctgggact     240
gggaaaacgg ccacatttgc catatcgatt ctgcagcaga ttgaattaga tctaaaagcc     300
acccaggcct tggtcctagc acccactcga gaattggctc agcagataca gaaggtggtc     360
atggcactag gagactacat gggcgcctcc tgtcacgcct gtatcggggg caccaacgtg     420
cgtgctgagg tgcagaaact gcagatggaa gctccccaca tcatcgtggg tacccctggc     480
cgtgtgtttg atatgcttaa ccggagatac ctgtccccca aatacatcaa gatgtttgta     540
ctggatgaag ctgacgaaat gttaagccgt ggattcaagg accagatcta tgacatattc     600
caaaagctca acagcaacac ccaggtagtt ttgctgtcag ccacaatgcc ttctgatgtg     660
cttgaggtga ccaagaagtt catgagggac cccattcgga ttcttgtcaa gaaggaagag     720
ttgaccctgg agggtatccg ccagttctac atcaacgtgg aacgagagga gtggaagctg     780
gacacactat gtgacttgta tgaaaccctg accatcaccc aggcagtcat cttcatcaac     840
acccggagga aggtggactg gctcaccgag aagatgcatg ctcgagattt cactgtatcc     900
gccatgcatg gagatatgga ccaaaaggaa cgagacgtga ttatgaggga gtttcgttct     960
ggctctagca gagttttgat taccactgac ctgctggcca gaggcattga tgtgcagcag    1020
gtttctttag tcatcaacta tgaccttccc accaacaggg aaaactatat ccacagaatc    1080
ggtcgaggtg gacggtttgg ccgtaaaggt gtggctatta acatggtgac agaagaagac    1140
aagaggactc ttcgagacat tgagaccttc tacaacacct ccattgagga aatgcccctc    1200

aatgttgctg acctcatc                                                    1218

<210>  28
<211>  951
<212>  DNA
<213>  Homo sapiens

<300>
<308>  GenBank/NM_006098
<309>  2003-04-07
<313>  (1)..(951)

<400>  28
atgactgagc agatgaccct tcgtggcacc ctcaagggcc acaacggctg ggtaacccag      60
atcgctacta ccccgcagtt cccggacatg atcctctccg cctctcgaga taagaccatc     120
atcatgtgga aactgaccag ggatgagacc aactatggaa ttccacagcg tgctctgcgg     180
ggtcactccc actttgttag tgatgtggtt atctcctcag atggccagtt tgccctctca     240
ggctcctggg atggaaccct gcgcctctgg gatctcacaa cgggcaccac cacgaggcga     300
tttgtgggcc ataccaagga tgtgctgagt gtggccttct cctctgacaa ccggcagatt     360
gtctctggat ctcgagataa aaccatcaag ctatggaata ccctgggtgt gtgcaaatac     420
actgtccagg atgagagcca ctcagagtgg gtgtcttgtg tccgcttctc gcccaacagc     480
agcaacccta tcatcgtctc ctgtggctgg gacaagctgg tcaaggtatg gaacctggct     540
aactgcaagc tgaagaccaa ccacattggc cacacaggct atctgaacac ggtgactgtc     600
tctccagatg gatccctctg tgcttctgga ggcaaggatg gccaggccat gttatgggat     660
ctcaacgaag gcaaacacct ttacacgcta gatggtgggg acatcatcaa cgccctgtgc     720
ttcagcccta accgctactg gctgtgtgct gccacaggcc ccagcatcaa gatctgggat     780
ttagagggaa agatcattgt agatgaactg aagcaagaag ttatcagtac cagcagcaag     840
gcagaaccac cccagtgcac ttccctggcc tggtctgctg atggccagac tctgtttgct     900

ggctacacgg acaacctggt gcgagtgtgg caggtgacca ttggcacacg c          951

<210>  29
<211>  963
<212>  DNA
<213>  Homo sapiens

<300>
<308>  genBank/NM_001436
<309>  2003-04-03
<313>  (1)..(963)

<400>  29
atgaagccag gattcagtcc ccgtgggggt ggctttggcg gccgaggggg ctttggtgac      60
cgtggtggtc gtggaggccg agggggcttt ggcgggggcc gaggtcgagg cggaggcttt     120
agaggtcgtg gacgaggagg aggtggaggc ggcggcggcg gtggaggagg aggaagaggt     180
ggtggaggct tccattctgg tggcaaccgg ggtcgtggtc ggggaggaaa aagaggaaac     240
cagtcgggga agaatgtgat ggtggagccg catcggcatg agggtgtctt catttgtcga     300
ggaaaggaag atgcactggt caccaagaac ctggtccctg gggaatcagt ttatggagag     360
aagagagtct cgatttcgga aggagatgac aaaattgagt accgagcctg gaaccccttc     420
cgctccaagc tagcagcagc aatcctgggt ggtgtggacc agatccacat caaaccgggg     480
gctaaggttc tctacctcgg ggctgcctcg ggcaccacgg tctcccatgt ctctgacatc     540
gttggtccgg atggtctagt ctatgcagtc gagttctccc accgctctgg ccgtgacctc     600
attaacttgg ccaagaagag gaccaacatc attcctgtga tcgaggatgc tcgacaccca     660
cacaaatacc gcatgctcat cgcaatggtg gatgtgatct ttgctgatgt ggcccagcca     720
gaccagaccc ggattgtggc cctgaatgcc cacaccttcc tgcgtaatgg aggacacttt     780
gtgatttcca ttaaggccaa ctgcattgac tccacagcct cagccgaggc cgtgtttgcc     840
tccgaagtga aaaagatgca acaggagaac atgaagccgc aggagcagtt gacccttgag     900

ccatatgaaa gagaccatgc cgtggtcgtg ggagtgtaca ggccaccccc caaggtgaag 960
aac 963

<210> 30
<211> 801
<212> DNA
<213> Homo sapiens

<300>
<308> GenBank/NM_000039
<309> 2003-04-03
<313> (1)..(801)

<400> 30
atgaaagctg cggtgctgac cttggccgtg ctcttcctga cggggagcca ggctcggcat 60
ttctggcagc aagatgaacc cccccagagc ccctgggatc gagtgaagga cctggccact 120
gtgtacgtgg atgtgctcaa agacagcggc agagactatg tgtcccagtt tgaaggctcc 180
gccttgggaa aacagctaaa cctaaagctc cttgacaact gggacagcgt gacctccacc 240
ttcagcaagc tgcgcgaaca gctcggccct gtgacccagg agttctggga taacctggaa 300
aaggagacag agggcctgag gcaggagatg agcaaggatc tggaggaggt gaaggccaag 360
gtgcagccct acctggacga cttccagaag aagtggcagg aggagatgga gctctaccgc 420
cagaaggtgg agccgctgcg cgcagagctc caagagggcg cgcgccagaa gctgcacgag 480
ctgcaagaga agctgagccc actgggcgag gagatgcgcg accgcgcgcg cgcccatgtg 540
gacgcgctgc gcacgcatct ggccccctac agcgacgagc tgcgccagcg cttggccgcg 600
cgccttgagg ctctcaagga gaacggcggc gccagactgg ccgagtacca cgccaaggcc 660
accgagcatc tgagcacgct cagcgagaag gccaagcccg cgctcgagga cctccgccaa 720
ggcctgctgc ccgtgctgga gagcttcaag gtcagcttcc tgagcgctct cgaggagtac 780
actaagaagc tcaacaccca g 801

<210> 31

<211> 816

<212> DNA

<213> Homo sapiens

<300>

<308> GenBank/NM_000039

<309> 2003-04-03

<313> (1)..(816)

<300>

<308> GenBank/BT007411

<309> 2003-03-13

<313> (1)..(816)

<400> 31

atggctgcca aagtgtttga gtccattggc aagtttggcc tggccttagc tgttgcagga 60

ggcgtggtga actctgcctt atataatgtg gatgctgggc acagagctgt catctttgac 120

cgattccgtg gagtgcagga cattgtggta ggggaaggga ctcattttct catcccgtgg 180

gtacagaaac caattatctt tgactgccgt tctcgaccac gtaatgtgcc agtcatcact 240

ggtagcaaag atttacagaa tgtcaacatc acactgcgca tcctcttccg gcctgtcgcc 300

agccagcttc ctcgcatctt caccagcatc ggagaggact atgatgagcg tgtgctgccg 360

tccatcacaa ctgagatcct caagtcagtg gtggctcgct ttgatgctgg agaactaatc 420

acccagagag agctggtctc caggcaggtg agcgacgacc ttacagagcg agccgccacc 480

tttgggctca tcctggatga cgtgtccttg acacatctga ccttcgggaa ggagttcaca 540

gaagcggtgg aagccaaaca ggtggctcag caggaagcag agagggccag atttgtggtg 600

gaaaaggctg agcaacagaa aaaggcggcc atcatctctg ctgagggcga ctccaaggca 660

gctgagctga ttgccaactc actggccact gcaggggatg gcctgatcga gctgcgcaag 720
ctggaagctg cagaggacat cgcgtaccag ctctcacgct ctcggaacat cacctacctg 780
ccagcggggc agtccgtgct cctccagctg ccccag 816

<210> 32
<211> 579
<212> DNA
<213> Homo sapiens

<300>
<308> GenBank/AF498970
<309> 2002-05-01
<313> (1)..(579)

<400> 32
atggctgcca tccggaagaa actggtgatt gttggtgatg gagcctgtgg aaagacatgc 60
ttgctcatag tcttcagcaa ggaccagttc ccagaggtgt atgtgcccac agtgtttgag 120
aactatgtgg cagatatcga ggtggatgga aagcaggtag agttggcttt gtgggacaca 180
gctgggcagg aagattatga tcgcctgagg cccctctcct acccagatac cgatgttata 240
ctgatgtgtt tttccatcga cagccctgat agtttagaaa acatcccaga aaagtggacc 300
ccagaagtca agcatttctg tcccaacgtg cccatcatcc tggttgggaa taagaaggat 360
cttcggaatg atgagcacac aaggcgggag ctagccaaga tgaagcagga gccggtgaaa 420
cctgaagaag gcagagatat ggcaaacagg attggcgctt ttgggtacat ggagtgttca 480
gcaaagacca aagatggagt gagagaggtt tttgaaatgg ctacgagagc tgctctgcaa 540
gctagacgtg ggaagaaaaa atctggttgc cttgtcttg 579